# EUROPEAN PATENT APPLICATION

(11) **EP 4 299 765 A1**
(43) Date of publication of application: **03.01.2024**
(21) Application number: 22759774.7
(22) Date of filing: 25.02.2022
(51) Int. Cl.: C12Q 1/70, C12N 15/11, C12Q 1/6844, C12Q 1/6851, C12Q 1/686, C12Q 1/6888, C12Q 1/689, G01N 33/543, G01N 33/569

(54) **METHOD FOR DETECTING AND QUANTIFYING NUCLEIC ACID FROM ENVIRONMENTAL SAMPLES**

(30) Priority: 26.02.2021 JP 2021029807; 22.04.2021 JP 2021072243; 29.10.2021 JP 2021177927
(71) Applicant: National University Corporation Hokkaido University, Sapporo-shi, Hokkaido 060-0808 (JP); Shionogi & Co., Ltd, Osaka-shi, Osaka 541-0045 (JP)
(72) Inventor: KITAJIMA, Masaaki, Sapporo-shi, Hokkaido 060-0808 (JP); IWAMOTO, Ryo, Osaka-shi, Osaka 541-0045 (JP); MASAGO, Yusaku, Osaka-shi, Osaka 541-0045 (JP); HAYASE, Shin, Osaka-shi, Osaka 541-0045 (JP); KATAYAMA ADACHI, Yuka, Osaka-shi, Osaka 541-0045 (JP)
(74) Representative: Vossius & Partner Patentanwälte Rechtsanwälte mbB
(86) International application number: PCT/JP2022/007766
(87) International publication number: WO 2022/181739

(57) **Abstract**

The present invention provides a method for quantitatively detecting virus-derived RNA and/or DNA in an environmental sample or a fecal sample with high sensitivity, and a kit for quantitatively detecting virus-derived RNA and/or DNA in an environmental sample or a fecal sample with high sensitivity.

## Description

### [TECHNICAL FIELD]

The present invention relates to methods for quantitatively detecting a nucleic acid (RNA and/or DNA) derived from a virus or a bacterium in an environmental sample or a fecal sample.

### [BACKGROUND ART]

During globalization, flow of people and things becomes active, and people are exposed to threat of infectious diseases caused by various viruses or bacteria. It is important to grasp occurrence of infectious diseases at an early stage, call for caution, and take countermeasures as soon as possible so as to minimize health damage and human and economic loss.

Examples of a method for grasping the occurrence of infectious diseases at an early stage include wastewater-based epidemiology (WBE). The wastewater-based epidemiology is to obtain epidemiological information in a treatment area by wastewater analysis, and its applicability to infectious diseases caused by viruses or bacteria such as COVID-19 has been suggested (Non-patent Document 1). Patients with infectious disease may be unevenly distributed in the same city due to cluster occurence in hospitals, welfare facilities, schools, offices, and the like where people live together. It is expected to grasp localization of infected people in the city and improve efficiency of inspection and distribution of medical resources to a region by implementing the wastewater-based epidemiology in society and utilizing the information.

In order to establish the method for grasping the occurrence of infectious diseases as described above at an early stage, a method for quantitatively detecting a nucleic acid derived from a virus or a bacterium from an environmental sample or the like with high sensitivity is required.

### [PRIOR ART DOCUMENT]

### [Non-patent Document]

[Non-patent Document 1] Sci. Total Environ., 739, 139076 (2020).

### [SUMMARY OF THE INVENTION]

### [PROBLEMS TO BE SOLVED BY THE INVENTION]

An object of the present invention is to provide a novel method for quantitatively detecting a nucleic acid derived from a virus or a bacterium in an environmental sample or a fecal sample.

### [MEANS FOR SOLVING THE PROBLEM]

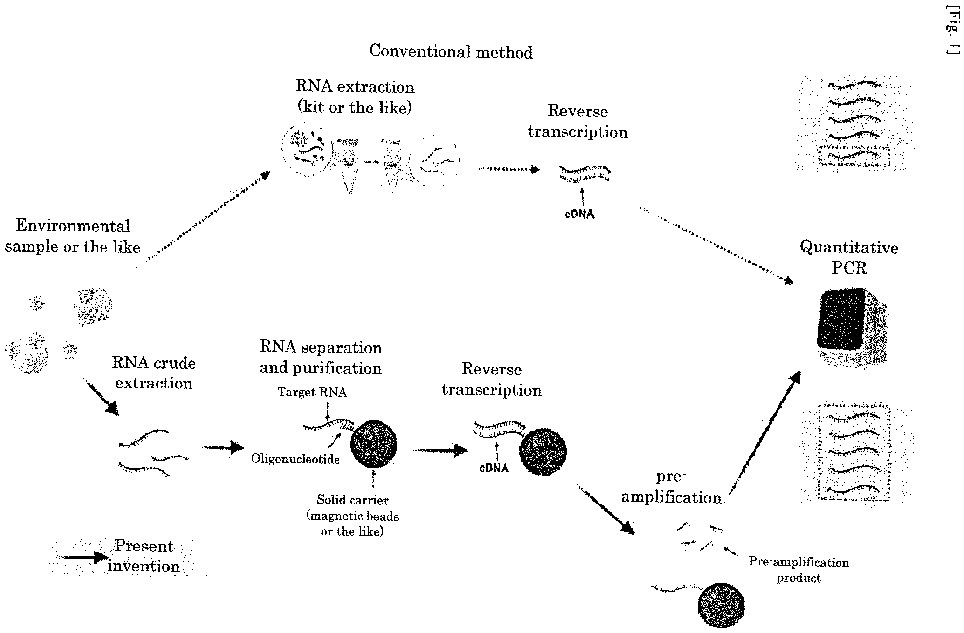

As a result of intensive studies, the present inventors have found a method for quantitatively detecting a nucleic acid (RNA and/or DNA) derived from a virus or a bacterium in an environmental sample or a fecal sample with high sensitivity (hereinafter, the method (I) of the present invention) (lower part of Fig. 1).

For example, for detection of a nucleic acid derived from a virus or a bacterium, particularly RNA detection, in a sample obtained from sludge, feces, or the like, commercial kits such as RNeasy PowerSoil Total RNA Kit, RNeasy PowerMicrobiome Kit (QIAGEN), Quick-DNA Fecal/Soil Microbe Kit (ZYMO RESEARCH), NucleoBond (Takara Bio Inc.), and ISOIL (NIPPON GENE Co., Ltd.) are generally used. However, since these kits have poor recovery efficiency of RNA derived from a virus or a bacterium and PCR inhibition occurs (PCR inhibition is severe particularly in a sample obtained from sludge), quantitative detection was impossible particularly when RNA derived from a virus or a bacterium was thinly contained in the sample (upper part of Fig. 1). Furthermore, since the time required is long, it is unsuitable when it is necessary to quantitatively detect RNA from many samples, such as grasping occurrence and spread of infectious diseases in cities. That is, the present inventors have found that the existing methods for detecting RNA derived from a virus or a bacterium from environmental samples and the like such as wastewater cannot meet the need for grasping the occurrence of infectious diseases at an early stage even in a situation where the number of infected people is small.

As described in Example 1, when virus-derived RNA in a wastewater sample was quantitatively detected using the method (I) of the present invention, there was almost no PCR inhibition and quantitativity was not lost, and RNA could be quantitatively detected with a high sensitivity of about 100 to 1000 times or more as compared with the known representative methods. Furthermore, it has been confirmed that quantitative detection is possible even when the virus-derived RNA is thinly contained in the sample.

In addition, RNA and/or DNA derived from a virus or a bacterium to be detected can be changed by changing the oligonucleotide on the solid carrier (in step (b1), the oligonucleotide functions as a reverse transcription primer) in step (b1) or (b2). For example, as described in Examples, it is conceivable to use, as the oligonucleotide, an oligonucleotide having an oligo (dT) sequence for detecting RNA derived from a virus such as SARS-CoV-2 or a bacterium, or an oligonucleotide having a base sequence specific to a nucleic acid sequence of a specific virus or bacterium for detecting RNA and/or DNA derived from the virus or bacterium. Furthermore, the time required is equal to or shorter than that of the method using the above commercial kits, and the cost can be also equal to or lower than them. In order to quantitatively detect RNA and/or DNA from many samples by social implementation, automation is required, but since RNA and/or DNA can be held on a solid carrier by the solid carrier used in step (b1) and/or (b2), centrifugation is unnecessary, and there is also an advantage that automation is easy.

Furthermore, the present inventors have found that it is useful to use polyaluminum chloride (hereinafter, also referred to as PAC.) and/or triiron tetraoxide when obtaining a concentrated product of an environmental sample or a fecal sample used in step (a) of the method (I) of the present invention or the like (hereinafter, the method (II) of the present invention). When this method is used, centrifugation is unnecessary when obtaining the concentrated product used in step (a) or the like from the environmental sample or the fecal sample, and there is an advantage that automation is easy.

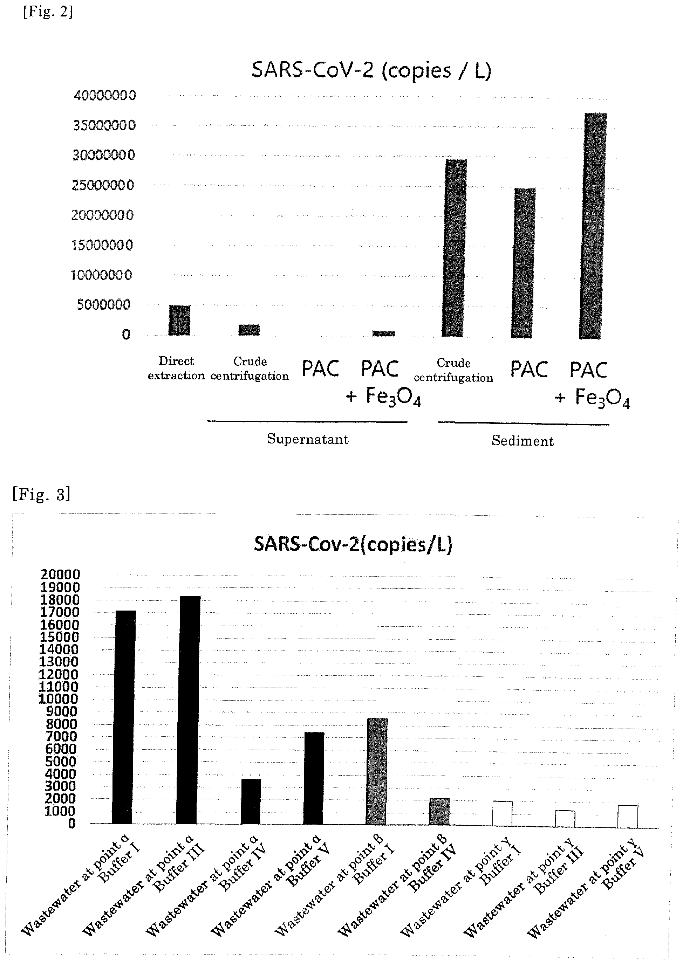

A flocculant used for water purification or the like neutralizes surface charges of fine particles to control attraction between the particles, thereby generating flocs. As the flocculant, an aluminum-based or iron-based inorganic flocculant is known. PAC and polyglutamic acid are frequently used as flocculants for the purpose of purifying water, but they are not generally used as reagents used for concentrating and recovering RNA and/or DNA from a wastewater sample. The present inventors have attempted RNA recovery using PGa21Ca (Nippon Poly-Glu Co., Ltd.) or PG-M (Nippon Poly-Glu Co., Ltd.) containing polyglutamic acid, but aggregates have been very strong, and quantitative detection has been difficult. On the other hand, when PAC was used, flocs were formed from the wastewater sample, and an amount of RNA sufficient for quantitative detection could be extracted from the flocs (concentrated products) (Fig. 2).

In addition, the present inventors have conducted studies on improving the recovery efficiency of RNA in a wastewater sample by combining PAC with various forms of iron (simple substance or compound). When a simple substance of iron (Fe) was used, iron powder was not dispersed in water and was not incorporated into flocs at all. When FG beads (registered trademark, Tamagawa Seiki Co., Ltd.), which are beads coated with poly GMA (glycidyl methacrylate), are used, FG beads are incorporated into flocs, but it is difficult to collect FG beads using a magnet due to weak magnetic force. Similarly, iron oxide (III) (FeaOs), iron sulfate (II) (FeSO₄), polyferric sulfate, and iron chloride (II) (FeCl₂) were also incorporated into flocs, but they were determined not to be used due to weak magnetism. On the other hand, triiron tetraoxide (Fe₃O₄) was incorporated into flocs and had magnetism, so that the flocs could be completely collected using a magnet. As a result, the recovery rate of RNA further increased as compared with the case of only PAC (Fig. 2), suggesting that accurate quantitative detection is possible.

In addition, the present inventors have found that quantitative detection efficiency of nucleic acid is remarkably excellent when a concentrated product of an environmental sample or a fecal sample is obtained using particularly high basicity PAC among PAC and the concentrated product is dissolved and lysed under neutral (particularly pH 7 to 8) conditions (hereinafter, the method of the present invention (III)).

A normal PAC has a basicity of about 45 to 55% (in the present specification, PAC having a basicity of about 45 to 55% is described as "normal PAC" as necessary.), but PAC having a basicity higher than that is classified into high basicity PAC (basicity is generally often defined as about 55 to 70%.) or an ultrahigh basicity PAC (basicity is generally often defined as about 70 to 90%.). (In the present specification, PAC with a basicity of about 55 to 90% is described as "high basicity PAC" as necessary.)

In general, the pH of urban wastewater is known to be 7 to 8. When normal PAC is used as a flocculant for wastewater purification, since the optimum pH of normal PAC is about 4.5 to 6, it is necessary to adjust the pH in the wastewater sample with an appropriate pH adjusting agent. On the other hand, the present inventors have focused on the high basicity PAC, and considered that the nucleic acid in wastewater can be efficiently detected from the width of its proper pH. From the following Examples, it has been found that it is possible to aggregate a sample only by adding high basicity PAC to a wastewater sample, and quantitative detection of nucleic acid thereafter can also be performed efficiently.

For example, in Water Res. 2014 Jan 1; 48: 375-86, it is described that high basicity PAC can be used as a flocculant in order to remove bacteriophage which is a model of pathogenic virus from river water, but it is neither described nor suggested that high basicity PAC can be used in a method for quantitatively detecting a nucleic acid derived from a virus or a bacterium from an environmental sample or the like. Furthermore, the conditions under which a nucleic acid derived from a virus or a bacterium in an environmental sample or a fecal sample can be quantitatively detected using PAC as a flocculant without inhibiting an enzymatic reaction system such as quantitative PCR have not been studied at all.

Since wastewater sediment (concentrated product) contains a large amount of proteins derived from a virus or a bacterium, contaminant proteins in wastewater, and the like in addition to nucleic acids derived from a virus or a bacterium, a strongly basic solution or the like is often used as a solution for dissolving and lysing them (in the present specification, it is described as "lysis and dissolution buffer".). However, when a strongly basic solution is used for dissolving and lysing wastewater sediment, there is a high possibility that the strongly basic solution serves to inhibit the PCR reaction downstream of the quantitative detection, and it is expected that the quantitative detection efficiency of nucleic acid will decrease.

Therefore, the present inventors have conducted studies on conditions such as the composition and pH of the lysis buffer, and found conditions under which a nucleic acid in a wastewater sample can be quantitatively detected with higher sensitivity. Specifically, it has been found that quantitative detection sensitivity of the nucleic acid in a wastewater sample is significantly improved by dissolving and lysing the wastewater sediment under a condition of pH 7 to 8.

That is, method (III) of the present invention using a step of obtaining a concentrated product of an environmental sample or a fecal sample in the presence of high basicity PAC (step (A1-a)) and a step of dissolving and lysing the concentrated product under a condition of pH 7 to 8 (step (A2-a)) is a method for efficiently detecting a nucleic acid from an environmental sample or a fecal sample without requiring a pH adjusting step.

Furthermore, the present inventors have studied the dissolution and lysis conditions in step (A2-a), and have found that the wastewater sediment is more efficiently dissolved and lysed under a condition of pH 7 to 8 when heating treatment at 50 to 60°C and protease addition are performed. From the following Examples, it was shown that RNA quantitative detection efficiency in the wastewater sample was further remarkably improved by the protease and heating treatment during dissolution and lysis.

In addition, the present inventors have found that when a lysis buffer containing 1 to 2% (w/v) of sodium lauryl sulfate (SDS) and 1 to 2 mmol/L dithiothreitol (DTT) is used in dissolving and lysing a concentrated product of an environmental sample or a fecal sample in step (A2-a), step (A2-b) or step (A2) described later, the recovery rate of nucleic acid is increased.

Unlike general silica beads, paramagnetic beads, particularly carboxyl group-modified paramagnetic beads, do not require a chaotropic salt such as guanidine, and can selectively bind nucleic acids in the presence of an organic solvent. Further, since the carboxyl group-modified paramagnetic beads can easily separate the bead to which the nucleic acid is bound and a wash buffer in which a substance inhibiting enzyme is dissolved and lysed by a magnet, the carboxyl group-modified paramagnetic beads hardly inhibit enzymatic reaction system.

From the following Examples, the present inventors have found that by using paramagnetic beads having the characteristics as a solid carrier in step (A3), the nucleic acid in the concentrated product, that is, in the wastewater sample, dissolved and lysed in step (A2-a), step (A2-b) or step (A2) described later can be efficiently separated, and then quantitative detection of nucleic acid can be performed with high sensitivity.

Furthermore, the present inventors have found that even when the step of concentrating an environmental sample or a fecal sample in the presence of high basicity PAC and dissolving and lysing a concentrated product of the sample is performed under basic conditions by a lysis buffer containing a surfactant and a reducing agent, the recovery rate of nucleic acid is increased although the efficiency is lower than that under neutral conditions (hereinafter, the method (IV) of the present invention).

Besides, the present inventors have found from the following Examples that a method including a step of concentrating an environmental sample or a fecal sample in the presence of PAC and dissolving and lysing a concentrated product of the sample under a condition of pH 7 to 11 is a highly sensitive nucleic acid quantitative detection method. (Hereinafter, the method (V) of the present invention)

Among viruses existing in wastewater, enveloped viruses such as SARS-CoV-2 have high binding to solids in wastewater, and it has been revealed that these viruses are likely to be localized in solids fraction (sediment) during wastewater centrifugation (Science of the Total Environment 763 (2021) 144587). Thus, the method (I) of the present invention or the like serves as an effective viral RNA extraction/detection means. It is known that PMMoV or the like having no envelope is likely to be present in a supernatant fraction at the time of wastewater precipitation, and an existing PEG precipitation method ((Science of the Total Environment 807 (2022) 150722) and manual for detecting novel coronavirus genes in wastewater (see Example 1)) or the like is also effective for the extraction and detection thereof.

On the other hand, there is no simple and inexpensive method that can quantitatively detect both enveloped viruses and non-enveloped viruses with high sensitivity. The present inventors have conducted intensive studies, and found the methods (III) to (V) of the present invention capable of efficiently recovering viruses present in both the solids (sediment) fraction and the supernatant fraction obtained at the time of wastewater precipitation operation.

Specifically, the quantitative detection efficiency of virus-derived RNA in wastewater was compared by the method (V) of the present invention, the method (I) of the present invention, and the PEG precipitation method or ultrafiltration method (Water Sci Technol (2021) 84 (8): 1997-2013) that are known methods, using a total of 12 samples obtained by sampling urban wastewater at different times at two different locations where SARS-CoV-2 patients exist in the watershed. In both the method (V) of the present invention and the method (I) of the present invention, the SARS-CoV-2 detection rate when performing qPCR was very high as 100% (12/12), whereas the detection rate was 41.67% (5/12) in the ultrafiltration method and 16.67% (2/12) in the PEG precipitation method. Furthermore, among the four methods, the detection amount of nucleic acid derived from SARS-CoV-2 by the method (V) of the present invention showed an average of 3.5 to 136.7 times that of other methods, and it was revealed that both the SARS-CoV-2 detection rate and the detection amount of nucleic acid are highest in the method (V) of the present invention. In addition, regarding the detection signal of the non-enveloped virus PMMoV as an internal control of wastewater, the detection rate when using the method (V) of the present invention is the highest, and the amount of nucleic acid is 3.8 to 67.9 times those by other methods on average. Thus, it can be said that the method (V) of the present invention is a method suitable for detecting both enveloped and non-enveloped viruses in wastewater.

The present invention relates to the following methods (I) of the present invention.
(I-1) A method for quantitatively detecting RNA and/or DNA derived from a virus or a bacterium in an environmental sample or a fecal sample, the method comprising the steps of:
   (a) extracting RNA and/or DNA from the sample or the concentrated product of the sample;
   (b1) performing reverse transcription using the RNA hybridized to an oligonucleotide on a solid carrier as a template to obtain an immobilized cDNA, and/or
   (b2) hybridizing the DNA to an oligonucleotide on a solid carrier; and
   (c) performing pre-amplification using the cDNA and/or DNA on the solid carrier as a template to obtain an amplification product in a liquid phase.
(I-2) The method according to (I-1), wherein the environmental sample is a wastewater sample, a water sample, an air sample, or a sample obtained from a solid surface.
(I-3) The method according to (I-1) or (I-2), wherein the number of cycles of pre-amplification is 1 to 14 cycles.
(I-4) The method according to any of (I-1) to (I-3), wherein the oligonucleotide is an oligonucleotide having an oligo (dT) sequence or a base sequence specific to a viral or bacterial nucleic acid to be detected.
(I-5) The method according to any of (I-1) to (I-4), wherein the solid carrier is magnetic beads.
(I-6) The method according to any of (I-1) to (I-5), wherein the virus is an RNA virus.
(1-7) The method according to any of (I-1) to (I-5), wherein the virus is a coronavirus, an influenza virus, a norovirus, a sapovirus, an Aichi virus, an adenovirus, an orthopneumovirus, or a polyomavirus.
(I-8) The method according to (I-7), wherein the coronavirus is SARS-CoV-2.
(I-9) The method according to any of (I-1) to (I-8), comprising a step of (d) quantifying the cDNA and/or DNA on the solid carrier by quantitative PCR using the amplification product obtained in step (c) as a template.
(I-10) The method according to (I-9), wherein Ct value of quantitative PCR using the amplification product as a template is 18 to 35.
(I-11) The method according to any of (I-1) to (I-10), comprising a step of obtaining the concentrated product of step (a) from the environmental sample or the fecal sample using polyaluminum chloride and/or triiron tetraoxide.

The present invention also includes the following.
(I-12) The method according to (I-1) to (I-11), wherein the RNA and/or DNA is thinly contained in the sample.
(I-13) The method according to (I-12), wherein the RNA and/or DNA is 8570 copies/L or less.
(I-14) The method according to (I-12), wherein the RNA and/or DNA is 1250 copies/L or more.

Further, the present invention also includes the following methods (II) and kits of the present invention.
(II-1) A method for obtaining a concentrated product of an environmental sample or a fecal sample using polyaluminum chloride and/or triiron tetraoxide, and extracting RNA and/or DNA from the concentrated product.
(II-2) A method for quantitatively detecting RNA and/or DNA derived from a virus or a bacterium in the sample, comprising the method according to (II-1).
(II-3) A kit for quantitatively detecting RNA and/or DNA derived from a virus or a bacterium in an environmental sample or a fecal sample, the kit comprising triiron tetraoxide and/or high basicity polyaluminum chloride.

Furthermore, the present invention also includes the following methods (III) and kit of the present invention.
(III-1) A method for quantitatively detecting RNA and/or DNA derived from a virus or a bacterium in an environmental sample or a fecal sample, the method comprising the steps of:
   (A1-a) obtaining a concentrated product of the sample in the presence of high basicity polyaluminum chloride;
   (A2-a) dissolving and lysing the concentrated product under a condition of pH 7 to 8;
   (A3) binding RNA and/or DNA in the concentrated product to a solid carrier and separating the RNA and/or DNA;
   (B) when RNA is separated in step (A3), performing reverse transcription using the RNA as a template to obtain cDNA; and
   (C) performing pre-amplification using the cDNA obtained in step (B) and/or the DNA obtained in step (A3) as a template to obtain an amplification product in a liquid phase.
(III-2) The method according to (III-1), wherein the environmental sample is a wastewater sample, a water sample, an air sample, or a sample obtained from a solid surface.
(III-3) The method according to (III-1) or (III-2), wherein the number of cycles of pre-amplification is 1 to 14 cycles.
(III-4) The method according to any of (III-1) to (III-3), wherein the solid carrier is magnetic beads.
(III-5) The method according to any of (III-1) to (III-4), wherein the virus is an RNA virus.
(III-6) The method according to any of (III-1) to (III-5), wherein the virus is a coronavirus, an influenza virus, a norovirus, a sapovirus, an Aichi virus, an adenovirus, or a polyomavirus.
(III-7) The method according to (III-6), wherein the coronavirus is SARS-CoV-2.
(III-8) The method according to any of (III-1) to (III-7), comprising a step of (D) quantifying the cDNA and/or DNA by quantitative PCR using the amplification product obtained in step (C) as a template.
(III-9) The method according to any of (III-1) to (III-8), wherein the step (A2-a) includes a step of reacting a protease.
(III-10) The method according to (III-10), wherein the protease is proteinase K.
(III-11) The method according to (III-9) or (III-10), wherein the step of reacting the protease is performed at 50°C to 60°C.
(III-12) The method according to any of (III-1) to (III-11), wherein the pH adjusting step in the step (A2-a) is a step of mixing a lysis buffer containing a surfactant and a reducing agent.
(III-13) The method according to (III-12), wherein the surfactant is sodium lauryl sulfate or cetyltrimethylammonium bromide.
(III-14) The method according to (III-12) or (III-13), wherein the concentration of the surfactant is 0.5 to 5 mass/volume percent.
(III-15) The method according to any of (III-12) to (III-14), wherein the reducing agent is dithiothreitol.
(III-16) The method according to (III-15), wherein the concentration of the reducing agent is 0.5 to 150 mmol/L.
(III-17) The method according to (III-16), wherein the high basicity polyaluminum chloride has a basicity of 60 to 75%.
(III-18) The method according to any of (III-1) to (III-17), wherein triiron tetraoxide is used in step (A1-a).
(III-19) The kit according to (II-3).
(III-20) The kit according to (III-19), further comprising a buffer at pH 7 to 11 for dissolving and lysing wastewater sediment.
(III-21) A method for quantitatively detecting RNA and/or DNA derived from a virus or a bacterium in an environmental sample or a fecal sample, the method comprising the steps of:
   (A1-a) obtaining a concentrated product of the sample in the presence of high basicity polyaluminum chloride;
   (A3-a) binding RNA and/or DNA in the concentrated product to carboxyl group-modified paramagnetic beads and separating the RNA and/or DNA;
   (B) when RNA is separated in step (A3-a), performing reverse transcription using the RNA as a template to obtain cDNA; and
   (C) performing pre-amplification using the cDNA obtained in step (B) and/or the DNA obtained in step (A3-a) as a template to obtain an amplification product in a liquid phase.

Further, the present invention also includes the following method (IV) of the present invention.
(IV-1) A method for quantitatively detecting RNA and/or DNA derived from a virus or a bacterium in an environmental sample or a fecal sample, the method comprising the steps of:
(A1-a) obtaining a concentrated product of the sample in the presence of high basicity polyaluminum chloride;
(A2-b) dissolving and lysing the concentrated product under a condition of pH 8 to 11;
(A3) binding RNA and/or DNA in the concentrated product to a solid carrier and separating the RNA and/or DNA;
(B) when RNA is separated in step (A3), performing reverse transcription using the RNA as a template to obtain cDNA; and
(C) performing pre-amplification using the cDNA obtained in step (B) and/or the DNA obtained in step (A3) as a template to obtain an amplification product in a liquid phase.

Furthermore, the present invention also includes the following methods (V) and kits of the present invention.
(V-1) A method for quantitatively detecting RNA and/or DNA derived from a virus or a bacterium in an environmental sample or a fecal sample, the method comprising the steps of:
   (A1) obtaining a concentrated product of the sample in the presence of polyaluminum chloride;
   (A2) dissolving and lysing the concentrated product under a condition of pH 7 to 11;
   (A3) binding RNA and/or DNA in the concentrated product to a solid carrier and separating the RNA and/or DNA; and
   (B) when RNA is separated in step (A3), performing reverse transcription using the RNA as a template to obtain cDNA,
      wherein the method optionally comprises
   (C) a step of performing pre-amplification using the cDNA obtained in step (B) and/or the DNA obtained in step (A3) as a template to obtain an amplification product in a liquid phase.
(V-2) The method according to (V-1), wherein the environmental sample is a wastewater sample, a water sample, an air sample, or a sample obtained from a solid surface.
(V-3) The method according to (V-1) or (V-2), wherein the number of cycles of pre-amplification is 1 to 14 cycles.
(V-4) The method according to any of (V-1) to (V-3), wherein the solid carrier is magnetic beads.
(V-5) The method according to any of (V-1) to (V-4), wherein the virus is an RNA virus.
(V-6) The method according to any of (V-1) to (V-5), wherein the virus is a coronavirus, an influenza virus, a norovirus, a sapovirus, an Aichi virus, an adenovirus, an orthopneumovirus, or a polyomavirus.
(V-7) The method according to (V-6), wherein the coronavirus is SARS-CoV-2.
(V-8) The method according to any of (V-1) to (V-7), comprising a step of (D) quantifying the cDNA and/or DNA by quantitative PCR using the amplification product obtained in step (C) or the cDNA obtained in step (B) and/or the DNA obtained in step (A3) as a template.
(V-9) The method according to any of (V-1) to (V-8), wherein the polyaluminum chloride has a final concentration of 0.001 to 0.01 mass/volume percent in step (A1).
(V-10) The method according to any of (V-1) to (V-9), wherein the polyaluminum chloride has a basicity of 50 to 70%.
(V-11) The method according to any of (V-1) to (V-10), wherein the step (A2) is performed under the condition of pH 7 to 8.
(V-12) The method according to any of (V-1) to (V-11), wherein the step (A2) includes a step of reacting a protease.
(V-13) The method according to (V-12), wherein the protease is proteinase K.
(V-14) The method according to (V-12) or (V-13), wherein the step of reacting the protease is performed at 50°C to 60°C.
(V-15) The method according to any of (V-1) to (V-14), wherein the pH adjusting step in the step (A2) is a step of mixing a lysis buffer containing a surfactant and a reducing agent.
(V-16) The method according to (V-15), wherein the reducing agent is dithiothreitol.
(V-17) The method according to (V-15) or (V-16), wherein the concentration of the reducing agent is 0.5 to 150 mmol/L.
(V-18) The method according to any of (V-15) to (V-17), wherein the surfactant is sodium lauryl sulfate or cetyltrimethylammonium bromide.
(V-19) The method according to any of (V-15) to (V-18), wherein the concentration of the surfactant is 0.5 to 5 mass/volume percent.
(V-20) The method according to any of (V-1) to (V-19), wherein triiron tetraoxide is further used in step (A1).
(V-21) The kit according to (II-3).
(V-22) A method for quantitatively detecting RNA and/or DNA derived from a virus or a bacterium in an environmental sample or a fecal sample, the method comprising the steps of:
   (A1-b) obtaining a concentrated product of the sample in the presence of 0.001 to 0.01 mass/volume percent of polyaluminum chloride;
   (A2) dissolving and lysing the concentrated product under a condition of pH 7 to 11;
   (A3-a) binding RNA and/or DNA in the concentrated product to carboxyl group-modified paramagnetic beads and separating the RNA and/or DNA; and
   (B) when RNA is separated in step (A3-a), performing reverse transcription using the RNA as a template to obtain cDNA,
      wherein the method optionally comprises a step of
   (C) performing pre-amplification using the cDNA obtained in step (B) and/or the DNA obtained in step (A3-a) as a template to obtain an amplification product in a liquid phase.
(V-23) A kit for quantitatively detecting RNA and/or DNA derived from a virus or a bacterium in an environmental sample or a fecal sample, the kit comprising polyaluminum chloride and a solid carrier.
(V-24) The kit according to (V-23), wherein the solid carrier is carboxyl group-modified magnetic beads.

### [EFFECT OF THE INVENTION]

The methods (I) to (V) of the present invention are methods for quantitatively detecting RNA and/or DNA derived from a virus or a bacterium in an environmental sample or a fecal sample with high sensitivity.

### [BRIEF DESCRIPTION OF THE DRAWINGS]

Fig. 1 shows a scheme of a conventional method and a method of the present invention (I). In the conventional method (upper part), only a small part of a sample can be provided for detection, but in the method (I) of the present invention (lower part), an equivalent amount of a sample that can be provided for detection can be maximized.
Fig. 2 shows observed concentrations of RNA derived from SARS-CoV-2 in sediments which are concentrated products obtained from PAC and/or triiron tetraoxide from a sample of a raw wastewater tank of a facility for accommodation and treatment of people with mild coronavirus infection and the like.
Fig. 3 shows observed concentrations of SARS-CoV-2 RNA in wastewater samples obtained from a plurality of urban wastewater.
Fig. 4 shows observed concentrations of PMMoV RNA in wastewater samples obtained from a plurality of urban wastewater.

### [MODE FOR CARRYING OUT THE INVENTION]

The term used in the present description is used in the meaning usually used in the art, unless specifically mentioned.

The "environmental sample" means a sample obtained from environment, excluding a sample obtained directly from a living body. Examples thereof include samples obtained from a wastewater, a sludge, a water, an air, a solid surface, a soil, a deposit, and the like. Preferably, it is a wastewater sample, a water sample, an air sample, or a sample obtained from a solid surface.

Examples of the "wastewater sample" include samples derived from a wastewater (for example, an inflow water, a treated water, a discharged water, and the like in a wastewater treatment plant, a septic tank, and the like), wastewater sludge (for example, a sludge itself obtained from a wastewater treatment plant, a dehydrated sludge, and the like), and the like.

Examples of the "water sample" include samples derived from drinking water, tap water, surface water, groundwater, wastewater, and the like.

Examples of the "air sample" include samples derived from air such as water, dirt, and dust in the atmosphere. For example, in addition to a filter of an air sampler in which air itself is filtered, dust of a filter of an air conditioner can be collected and used.

Examples of the "sample obtained from a solid surface" include samples derived from moisture, oil, dirt, dust, or the like attached to a solid surface other than a living body. For example, a deposit on the surface of a doorknob or a button of an elevator can be collected and used.

The "fecal sample" means a sample derived from feces of an animal such as a human, a dog, a cat, a monkey, a rabbit, a cow, a horse, a chicken, a hamster, a rat, or a mouse, and is preferably human feces.

The "virus" of "RNA and/or DNA derived from a virus or a bacterium" detected by the method of the present invention is not particularly limited, but is preferably an RNA virus, particularly preferably a pathogenic RNA virus infecting humans.

Examples of the RNA virus include viruses having an envelope that is a membrane composed of a lipid bilayer such as a coronavirus, an influenza virus, a human immunodeficiency virus, a hepatitis C virus, a Japanese encephalitis virus, an orthopneumovirus, and a dengue virus; and non-enveloped viruses such as a norovirus, a rotavirus, a rhinovirus, a sapovirus, an Aichi virus, and a pepper mild mottle virus.

Examples of the DNA virus include enveloped viruses such as poxvirus and herpes virus; and non-enveloped viruses such as adenovirus, papillomavirus, polyomavirus, and parvovirus.

In the method of the present invention, the "virus" is preferably a coronavirus, an influenza virus, a norovirus, a sapovirus, an Aichi virus, an adenovirus, an orthopneumovirus, or a polyomavirus, and further preferably a coronavirus, an influenza virus, a norovirus, an orthopneumovirus, a sapovirus, or an Aichi virus. The coronavirus is particularly preferably SARS-CoV-2, MERS-CoV, SARS-CoV, or the like.

The "bacterium" in "RNA and/or DNA derived from a bacterium" as detected by the method of the present invention means eubacteria or archaea. The bacterium is preferably an aerobic bacterium, and more preferably a pathogenic aerobic bacterium infecting humans.

Examples of the aerobic bacterium include a Salmonella, a Shigella, a Vibrio parahaemolyticus, a Vibrio cholerae, a Staphylococcus aureus, an Enterohemorrhagic Escherichia coli, a Streptococcus pneumoniae, a Salmonella typhimurium, a Haemophilus influenzae, a Pseudomonas aeruginosa, and the like, which are causative bacteria of bacterial food poisoning.

Examples of the "solid carrier" include columns, beads, test tubes, microtiter dishes, solid particles, inner walls of microtubes, membranes, and the like. Preferably, the solid carrier is magnetic beads. In the method (III) of the present invention, the solid carrier is further preferably carboxyl group-modified paramagnetic beads, carboxyl group-modified diamagnetic beads, or a capillary with a carboxyl-modified inner wall. The carboxyl group-modified paramagnetic beads mean paramagnetic beads whose surface is coated with a carboxyl group, and examples thereof include RNAClean XP (BECKMAN COULTER) and the like.

The "polyaluminum chloride (PAC)" is a compound whose chemical name is basic aluminum chloride (CAS No.: 1327-41-9) and whose molecular formula is [Al₂(OH)ₙCl_{6·n}]m (0 < n < 6, m ≤ 10). The basicity is represented by n/6 × 100 (%). Some sulfate ions (SO₄²⁻) may be introduced as an active ingredient.

The "high basicity polyaluminum chloride (high basicity PAC)" means a PAC with a basicity of about 55 to 90%. The high basicity PAC is preferably a PAC with a basicity of 60 to 80%, and more preferably a PAC with a basicity of 60 to 75%.

The "lysis buffer" means a solution for dissolving and lysing (suspending) nucleic acids derived from a virus or bacterium, proteins derived from a virus or a bacterium, and contaminant proteins in wastewater, contained in wastewater sediment. The lysis buffer contains one or more surfactants and one or more reducing agents, and a form thereof is not particularly limited. The form may be a dry form or a solution form. As the solvent, water is usually used, but a mixed solvent with another solvent may be used as necessary. The lysis buffer includes all generally used lysis buffers for nucleic acid or protein extraction, and is not particularly limited, and examples thereof include lysis buffers including buffers, surfactants, salts, chelating agents, and reducing agents, solution1 of a DNA extraction kit (Cat. No. 144-200, SHIMADZU RIKA CORPORATION), or the like. The lysis buffer is preferably Lysis buffer I to Lysis buffer V described in Examples. The lysis buffer is more preferably Lysis buffer I (pH 7.5 to pH 11.0) described in Example 8.

The "surfactant" is a generic term for substances having a structure having both a hydrophilic portion and a hydrophobic portion in the molecule. Examples of the surfactant include anionic surfactants such as alkyl sulfates, alkyl ether sulfates, docusate, sulfonate fluorosurfactants, alkyl benzene sulfonates, alkyl aryl ether phosphates, alkyl ether phosphates, alkyl carboxylates, sodium lauroyl sarcosine, carboxylate fluorosurfactants, sodium cholate, and sodium deoxycholate; cationic surfactants such as ethyltrimethylammonium bromide, hexadecyltrimethylammonium bromide, tetradecyltrimethylammonium bromide, and cetyltrimethylammonium bromide (CTAB); amphoteric surfactants such as betaines and alkylamino fatty acid salts; and nonionic surfactants such as nonylphenoxypolyethoxyethanol (NP-40), polyoxyethylene sorbitan monooleate (Tween (trademark registration) 80), and polyoxyethylene p-t-octylphenol (Triton X-100 (trademark registration)). The surfactant is preferably an alkyl sulfate or CTAB, and is more preferably lithium dodecyl sulfate (LiDS), sodium lauryl sulfate (sodium dodecyl sulfate) (SDS) or CTAB.

The "reducing agent" means a chemical species that donates electrons to another chemical species. Examples of the reducing agent include dithiothreitol (DTT), 2-mercaptoethanol (2-Me), tris(2-carboxyethyl)phosphine (TCEP), tributylphosphine, iodoacetamide, iron(II), N-acetyl-L-cysteine (NALC), and the like. The reducing agent is preferably DTT. The reducing agents used in the methods (III) to (V) of the present invention also include those which do not originally exhibit a reducing action but exhibit a reducing action in the reagent.

The "protease" is not particularly limited as long as it has an ability to degrade proteins to such an extent that nucleic acids derived from a virus or a bacterium in an environmental sample or a fecal sample are dissolved and lysed, and examples thereof include proteinase K, trypsin, chymotrypsin, subtilisin and the like belonging to serine protease, pepsin and cathepsin D and the like belonging to aspartic protease, papain, cathepsin, caspase, calpain and the like belonging to cysteine protease, and the like. One or two or more of these can be used, but the protease is preferably proteinase K having a wide substrate specificity of the protein and maintaining the activity even in the presence of a chelating agent or a denaturant.

Hereinafter, specific embodiments of the present invention will be described, but the present invention is not limited thereto.

### (Invention related to the method (I) of the present invention)

In step (a), RNA and/or DNA derived from a virus or a bacterium in an environmental sample or a fecal sample is extracted from the sample or a concentrated product of the sample. The sample may be used as it is in the step of extracting RNA and/or DNA, or may be concentrated and used by a method known in the art. Examples of a method for concentrating a sample generally include filtration, sedimentation, ultracentrifugation, crude centrifugation, and the like. For example, when it is known that RNA and/or DNA derived from a virus or a bacterium is present at a high rate in a solid fraction in a sample, highly sensitive RNA and/or DNA detection can be achieved by concentrating only the solid fraction.

As a method for extracting RNA and/or DNA, a method known in the art using a commercially available kit, a conventional reagent/device, or the like can be used. Examples thereof include a method using a spin column having a silica membrane, a phenol/chloroform extraction method, and the like. Furthermore, RNA and/or DNA may be purified using NucleoSpin (trademark registration) RNA Clean-up (Takara Bio Inc.) or the like.

A solid sample or a concentrated product can be crushed, added with a nonpolar solvent such as phenol or chloroform, and then used in the above method.

In step (b1) or (b2), the RNA and/or DNA extracted in step (a) is hybridized with an oligonucleotide immobilized on a solid carrier in order to improve recovery efficiency of RNA and/or DNA. When RNA and/or DNA derived from a virus or a bacterium is a double strand, the RNA and/or DNA is dissociated into single strands before hybridization. The dissociation from a double strand into single strands can be performed by a method known in the art. Step (b1) is performed when RNA is quantitatively detected, and step (b2) is performed when DNA is quantitatively detected. Steps (b1) and (b2) can be performed sequentially or simultaneously after step (a) using the same sample.

The oligonucleotide may have a sequence useful for recovery of RNA and/or DNA derived from a virus or a bacterium, and an oligonucleotide known in the art can be used. Examples of the oligonucleotide include an oligonucleotide having an oligo (dT) sequence, an oligonucleotide having a base sequence specific to a viral nucleic acid to be detected, and the like. For example, when RNA derived from a virus (for example, coronavirus) or a bacterium having a poly (A) tail is extracted, an oligo (dT) primer can be used. The length of the oligonucleotide is, for example, 17 to 30 bases.

The "oligonucleotide having a base sequence specific to a viral or bacterial nucleic acid to be detected" is an oligonucleotide having at least 70% or more, preferably 80% or more, more preferably 90% or more, and most preferably 95% or more homology with a complementary sequence of a partial sequence of a viral or bacterial nucleic acid to be detected, and examples thereof include a sequence that is not present in a complementary sequence of RNA and/or DNA derived from another organism, virus, or the like that may be present in a sample. Here, the homology shows similarity as a score, for example, by using BLAST, a search program using an algorithm developed by Altschul et al. (The Journal of Molecular Biology, 215, 403-410 (1990).).

When the target for quantitative detection is viral RNA or bacterial RNA, a reverse transcription reaction is performed as described in step (b1). At this time, the oligonucleotide immobilized on the solid carrier functions as a reverse transcription primer. Thus, cDNA immobilized on the solid carrier is obtained.

In step (c), the cDNA on the solid carrier obtained in step (b1) and/or the DNA hybridized to the oligonucleotide on the solid carrier in step (b2) is used as a template to obtain a pre-amplified product in a liquid phase.

The reverse transcription in step (b1) and the pre-amplification in step (c) may be performed separately in two steps, or may be performed at once as one step.

The number of cycles of pre-amplification is, for example, 1 to 14, 6 to 14, 6 to 10, or 10 to 14.

In step (b1) or (b2), RNA and/or DNA derived from a virus or a bacterium is hybridized with an oligonucleotide on a solid carrier from a sample or a concentrated product thereof, whereby the concentration ratio can be increased. Further, in step (c), since a product of pre-amplification is generated in the liquid phase, only the amplification product can be brought into quantitative PCR of step (d), and a cDNA cleavage step from a solid carrier is unnecessary in quantitative measurement. Furthermore, since the immobilized (c)DNA can be reused as a template, the (c)DNA can be used for PCR for another target, PCR amplification for sequence analysis, or the like (for example, an oligonucleotide on a solid carrier can be used multiple times for different purposes, for example, in the first quantitative PCR, the amount of virus is quantified using a part of the oligonucleotide as a target, and then a mutant strain of virus or bacteria is detected by PCR or sequence analysis using another site of the oligonucleotide as a target), which is very useful in reducing costs, tasks, and time in automation for social implementation.

In step (d), quantitative PCR (qPCR, real-time PCR) is performed using the pre-amplification product obtained in step (c) as a template. The quantitative PCR can be performed by a method known in the art, and can be performed using a commercially available kit, a conventional reagent/device, or the like.

The detection cycle threshold (Ct value) of quantitative PCR using the pre-amplification product as a template is, for example, 18 to 35.

As one of the characteristics of the method of the present invention, quantitative detection is possible even when RNA and/or DNA derived from a virus or a bacterium is thinly contained in a sample. Since the pre-amplification product is used as a template in the quantitative PCR, even if the amount of RNA and/or DNA derived from a virus or a bacterium is so small that it cannot be quantitatively detected by a conventional method, in the method of the present invention, an equivalent amount of a sample that can be subjected to detection increases, and sufficient quantitative detection can be performed. In the conventional method described in Examples, the lower limit of detection of RNA and/or DNA was 8570 copies/L, but in the method of the following (Method 2), the lower limit of detection was 1250 copies/L. It is expected that the method of the present invention can perform quantitative detection even when RNA and/or DNA in a sample is further diluted. That is, the method of the present invention is useful for early recognition of the occurrence of infectious diseases because quantitative detection is expected even in a sample in a situation where the number of infected people is small.

Also, as another embodiment of the method of the present invention, as an RNA and/or DNA quantitative method using a pre-amplification product as a template, other known methods can be used instead of quantitative PCR. Specific examples thereof include CONAN (Cas3 Operated Nucleic Acid detectioN) method (DOI number: 10.1101/2020.06.02.20119875), SHERLOCK (Specific High Sensitivity Enzymatic Reporter UnLOCKing) method (DOI number: 10.1101/2020.05.04.20091231), LAMP (Loop-mediated Isothermal Amplification) method, and RPA (Recombinase Polymerase Amplificaition) method, and the like.

Among the methods of the present invention, examples of a method using a wastewater sample include the following. In the case of detecting RNA viruses (in particular, enveloped viruses such as SARS-CoV-2) in wastewater, it has been reported that RNA viruses are present in a solid fraction at a high rate (for example, Sci.Total Environ., 763, 144587 (2020).). Therefore, in the case of detecting enveloped viruses, it is preferable to extract RNA and/or DNA from the solid fraction.

### (Method 1)

Step (a): The entire amount of sludge or sediment in a wastewater sample (40 to 50 mL) is crushed using, for example, a Lysis buffer of RNeasy PowerSoil Kit (QIAGEN) and phenol/chloroform to elute RNA and/or DNA. The aqueous layer is collected, 500 µL is transferred to a 2 mL tube, and the same amount of Binding Buffer (example: 20 to 100 mmol/L Tris-HCl, pH 7.5, 1.0 M LiCl, 1 to 2 mmol/L EDTA) is added. In order to resolve RNA conformation, the resulting solution is incubated at 65°C for 2 to 5 minutes and immediately ice-cooled.

Step (b1): A washed solid carrier (for example, Dynabeads (registered trademark) Oligo dT₂₅ (Veritas Corporation)) resuspended in 200 µL of Binding Buffer is added. Annealing is performed at room temperature for 5 to 10 minutes while rotating the mixture with a rotator. After standing for 2 minutes, the supernatant is removed. 500 µL to 1 mL of Washing Buffer I (example: 10 to 20 mmol/L Tris-HCl, pH 7.5, 0.15 M LiCl, 1 mmol/L EDTA) is added thereto for washing while pipetting 2 or 3 times. After standing for 2 minutes, the supernatant (Washing Buffer I) is completely removed. 500 µL to 1 mL of Washing Buffer II (example: 10 to 20 mmol/L Tris-HCl, pH 7.5, 0.15 M LiCl, 1 mmol/L EDTA) is added thereto for washing while pipetting 2 or 3 times. After standing for 2 minutes, the supernatant (Washing Buffer II) is completely removed. 500 µL to 1 mL of Law-Salt Buffer (example: 10 to 20 mmol/L Tris-HCl, pH 7.5, 0.15 M LiCl, 1 mmol/L EDTA) is added thereto for washing while pipetting 2 or 3 times. After standing for 2 minutes, the supernatant (Law-Salt Buffer) is completely removed. Thereafter, the resulting solution is resuspended in 10 µL of 10 mmol/L Tris-HCl (pH 7.5) or water (RNase Free water).

For example, cDNA is synthesized by mixing 10 µL of a suspended solid carrier and 10 µL of a reverse transcription reaction reagent mix using High Capacity cDNA Reverse Transcription Kit (ThermoFisher Scientific). The oligonucleotide on the solid carrier functions as a reverse transcription primer. The reaction is carried out at 25°C for 10 minutes, 37°C for 120 minutes, and 85°C for 5 minutes (reverse transcription step).

Step (c): For example, using KOD One (registered trademark) (TOYOBO CO., LTD.), reaction is performed at 94°C for 30 seconds, 55°C for 30 seconds, and 72°C for 1 minute in 1 to 14 cycles (pre-amplification step).

Step (d): For example, qPCR is performed using QuantiTect (trademark) Probe PCR Kit (QIAGEN) using a pre-amplification product as a template.

In Method 1, the entire amount of RNA eluate can be brought into step (b1), and in principle, quantitative detection is considered possible as long as even one copy of RNA can be captured by a solid carrier.

In addition, the present inventors have noticed usefulness of the "pre-amplification step" in Method 1, and found that the following two methods can also be used for quantitative detection although detection sensitivity is inferior to that of Method 1.

### (Method 2)

Step (a): Polyethylene glycol (4 g) and sodium chloride (0.9 g) are added to a wastewater sample (40 to 50 mL), and the mixture is allowed to stand overnight and then centrifuged (12,000 g, 30 min to 1 h). After discarding the supernatant after centrifugation, RNA is extracted from a part (140 µl) of pellet resuspension, for example, using QIAamp (registered trademark) Viral RNA Mini Kit (QIAGEN).

Steps (b1) and (c): For example, after performing reverse transcription (2 hours) using High Capacity cDNA Reverse Transcription Kit (ThermoFisher Scientific), pre-amplification is performed using cDNA as a template (two steps). In this step, for example, reverse transcription and pre-amplification can also be performed in one step using iScript Explore One-Step RT and PreAmp Kit (Bio-Rad Laboratories, Inc.). Alternatively, a reverse transcription reaction can be performed by iScript (registered trademark) Advanced cDNA Synthesis Kit for RT-qPCR (Bio-Rad Laboratories, Inc.) or Reliance Select cDNA Synthesis Kit (BIORAD), and pre-amplification can be performed by BIOTAQ (registered trademark) HS (Meridian BIOSCIENCE). The pre-amplification conditions are the same as those in Method 1.

Step (d): qPCR is performed using the pre-amplification product as a template in the same manner as in Method 1.

### (Method 3)

Step (a): A wastewater sample (40 to 50 mL) is centrifuged (2,500 to 5,000 g, 10 to 30 min) (crude centrifugation). The entire amount of the resulting sediment suspension is charged into, for example, a 2 mL tube of RNeasy PowerMicrobiome Kit (QIAGEN). In step (a), steps after column purification can be automated using QIAcube (QIAGEN).

Steps (b1) and (c): Reverse transcription and pre-amplification are performed in the same manner as in Method 2.

Step (d): qPCR is performed using the pre-amplification product as a template in the same manner as in Method 1.

That is, the following method is adopted.
A method for quantitatively detecting RNA and/or DNA derived from a virus or a bacterium in an environmental sample or a fecal sample, the method comprising the steps of:
   (a) extracting RNA and/or DNA from the sample or a concentrated product of the sample;
   (bc-1) performing reverse transcription and pre-amplification of the extracted RNA, and/or
   (bc-2) performing pre-amplification of the extracted DNA; and
   (d) quantifying cDNA and/or DNA by quantitative PCR using the amplification product obtained in step (bc-1) and/or (bc-2) as a template.

Among the methods of the present invention, in a method using a wastewater sample, pepper mild mottle virus (PMMoV) can be used as an internal control. Using the method of the present invention, it is also possible to separately quantitatively detect a virus to be measured (example: SARS-CoV-2) and PMMoV. In addition, an oligonucleotide that hybridizes to a virus to be measured and an oligonucleotide that hybridizes to PMMoV are immobilized on the same solid carrier and used in step (b1) or (b2), whereby the virus to be measured and PMMoV can be simultaneously captured from the wastewater sample. Thereafter, step (b1) or (b2) to step (c) can also be performed in the same container by adding primers corresponding thereto or the like.

### (Invention related to the method (II) of the present invention)

The present invention includes a method for obtaining a concentrated product of an environmental sample or a fecal sample using PAC and/or triiron tetraoxide, and extracting RNA and/or DNA from the concentrated product. Further, the present invention also includes a method for quantitatively detecting RNA and/or DNA derived from a virus or a bacterium in an environmental sample or a fecal sample, the method including a step of obtaining a concentrated product of the environmental sample or the fecal sample using PAC and/or triiron tetraoxide, and extracting RNA and/or DNA from the concentrated product. PAC and/or triiron tetraoxide are used in the step of obtaining a concentrated product of the environmental sample or the fecal sample, and the methods described above or known in the art can be used in the subsequent step.

PAC is added in an amount of, for example, 1 µL to 40 µL, more preferably 2 µL to 10 µL, and further preferably 4 µL per 40 mL of the environmental sample or the fecal sample.

As the PAC, high basicity PAC is particularly preferable. The high basicity PAC is preferably a PAC with a basicity of 60 to 80%, and more preferably a PAC with a basicity of 60 to 75%. Examples thereof include PAC 700A (trademark registration) (Taki Chemical Co., Ltd.), PAC 300A (trademark registration) (Taki Chemical Co., Ltd.), Alfine 83 (TAIMEI CHEMICALS CO., LTD.), PAC#100W, PAC#100H (Asada Chemical Industry Co., Ltd.), and the like.

Triiron tetraoxide is added in an amount of, for example, 0.01 to 1.0 g, more preferably 0.01 to 0.1 g, and further preferably 0.02 g per 40 mL of the environmental sample or the fecal sample.

As the step of extracting RNA and/or DNA from the concentrated product of the environmental sample or the fecal sample, a method known in the art can be used as described in the description of the above step (a).

In the case of a concentrated product containing PAC, RNA and/or DNA can be efficiently recovered particularly by a method using a basic buffer or a method using Lysis buffer of RNeasy PowerSoil Total RNA Kit (QIAGEN) and phenol/chloroform. Examples of the basic buffer include glycine buffer and the like.

The present invention includes a kit for quantitatively detecting RNA and/or DNA derived from a virus or a bacterium in an environmental sample or a fecal sample, the kit comprising triiron tetraoxide. Specifically, examples of the kit include a kit for quantitatively detecting RNA and/or DNA derived from a virus or a bacterium in an environmental sample or a fecal sample which contains triiron tetraoxide and is used in the method (II) of the present invention or the like.

### (Invention related to the method (III) of the present invention)

The present invention includes a method for quantitatively detecting RNA and/or DNA derived from a virus or a bacterium in an environmental sample or a fecal sample, the method including the steps of: obtaining a concentrated product of the sample in the presence of high basicity PAC; dissolving and lysing the concentrated product under a condition of pH 7 to 8; and binding the RNA and/or DNA to a solid carrier and separating the RNA and/or DNA.

In step (A1-a), a concentrated product of an environmental sample or a fecal sample is obtained in the presence of a high basicity PAC. The method can be performed in the same manner as the "method for concentrating an environmental sample or a fecal sample" described in the description of the above step (a) except that it is in the presence of high basicity PAC.

The preferred basicity of the high basicity PAC and the preferred high basicity PAC concentration per environmental sample or fecal sample is similar to the (high basicity) PAC as described in the method (II).

Triiron tetraoxide may be added as in the method (II) of the present invention.

In step (A2-a), the concentrated product obtained in step (A1-a) is dissolved and lysed under a condition of pH 7 to 8.

In the pH adjusting step in step (A2-a), for example, the pH may be adjusted by mixing the concentrated product with a lysis buffer described later, or may be adjusted by an appropriate pH adjusting agent after dissolving and lysing the concentrated product in a basic buffer or an acidic buffer.

The mixing ratio (volume ratio) between the environmental sample or the fecal sample and the lysis buffer is not particularly limited, but can be, for example, 1 : 1000 to 1 : 10, and preferably 1 : 100 to 1 : 10.

The pH of the lysis buffer is not particularly limited as long as it is the pH of a solution that can dissolve and lyse nucleic acids derived from a virus or a bacterium, proteins derived from a virus or a bacterium and contaminant proteins in wastewater contained in wastewater sediment, but the pH of the lysis buffer is preferably 7 to 11 and particularly preferably 7.5 to 9. The pH can be adjusted by a pH adjusting agent or a buffer.

Examples of the pH adjusting agent and the buffer include phosphate buffer, Tris hydrochloride buffer, Tris acetate buffer, HEPES buffer, MOPS buffer, MES buffer, sodium acetate buffer, sodium citrate buffer, glycine buffer, and the like. The buffer is not particularly limited as long as it promotes elution of nucleic acid and is suitable for nucleic acid preservation, but Tris hydrochloride buffer (100 mmol/L Tris-HCl, 10 mmol/L EDTA) adjusted to an arbitrary pH is particularly preferable.

The concentration of surfactant in the lysis buffer is preferably 0.5 to 5 mass/volume percent ((% (w/v)), more preferably 1 to 3% (w/v), and particularly preferably 1 to 2% (w/v).

Step (A2-a) may further include a step of reacting a protease. The concentration of the protease in a suspension with the lysis buffer is not particularly limited depending on the type of the protease, but is preferably 0.14 to 150 U/mL, and further preferably 1 to 45 U/mL. In particular, when a reagent for nucleic acid elution contains proteinase K as a protease at the above-described concentration, the above-described effect is remarkably obtained.

When step (A2-a) includes a step of reacting a protease, heating treatment may be performed. The temperature is preferably 37°C to 70°C, more preferably 50°C to 60°C, and particularly preferably 56°C. The time for the heating treatment is not particularly limited, and is, for example, 5 to 60 minutes, preferably 5 to 30 minutes, and more preferably 5 to 15 minutes.

When step (A2-a) does not include the step of reacting a protease, the temperature of the lysis buffer can be 25 to 70°C.

The concentration of the reducing agent in the lysis buffer is not particularly limited, and is, for example, 0.5 to 150 mmol/L, preferably 1 to 5 mmol/L in the case of DTT, and preferably 10 to 150 mmol/L in the case of β-mercaptoethanol.

The lysis buffer may further contain various components. It may contain components necessary for a subsequent nucleic acid amplification reaction, for example, enzymes, salts such as magnesium salts and potassium salts, saccharides such as trehalose, sugar alcohols such as glycerol, nucleic acid components, organic solvents, and the like. The organic solvent has an effect of promoting the dissolution and lysis of RNA and/or DNA derived from a virus or a bacterium, and is particularly acceptable. Examples of the organic solvent include dimethyl sulfoxide (DMSO). In addition, an RNase inhibitor or the like can be added as long as the nucleic acid amplification reaction is not disturbed.

In step (A3), RNA and/or DNA in the concentrated product obtained in step (A1-a) is bound to a solid carrier having nucleic acid binding ability and separated. As a method for separating RNA and/or DNA from the solid carrier, a known method can be used. Examples thereof include a method of fixing the solid carrier using a magnet.

Here, binding means attaching a nucleic acid to a solid carrier. There is an advantage that a wash operation of a solid carrier to which RNA and/or DNA is strongly bound is easy, and automation by a robot or the like also becomes easy. The wash operation refers to an operation of removing an organic component not bound to a solid carrier using 70% ethanol or the like.

As the solid carrier, magnetic beads are preferable. The magnetic beads refer to beads having magnetic particles as a core. Examples thereof include magnetic silica beads, magnetic cellulose beads, and the like. The magnetic beads are preferably carboxyl group-modified paramagnetic beads. As with the carboxyl group-modified paramagnetic beads, beads having a property of reversibly binding a nucleic acid to the bead surface are included in the solid carrier used in the method of the present invention.

In step (B), when RNA is separated in step (A3), reverse transcription is performed using the RNA as a template to obtain cDNA. When DNA is quantitatively detected, step (B) does not need to be performed. These steps can be performed by methods known in the art.

In step (C), pre-amplification is performed using the cDNA obtained in step (B) and/or the DNA obtained in the steps up to step (A3) without passing through step (B) as a template, and an amplification product is obtained in a liquid phase. These steps can be performed by methods known in the art.

The pre-amplification step is essential when RNA and/or DNA derived from a virus or a bacterium is thinly contained in the sample. On the other hand, in a case where RNA and/or DNA derived from a virus or a bacterium is present at a high concentration, for example, in a case where a virus is detected from wastewater in a facility where there are very many virus-infected people, the virus in the wastewater can be efficiently detected without passing through the pre-amplification step.

In step (D), quantitative PCR is performed using the pre-amplification product obtained in step (C) as a template in the same manner as in the above step (d).

### (Method 4)

Step (A1-a): High basicity PAC (1 µL) is added to a wastewater sample (10 to 20 mL), and the mixture is mixed and then stirred (50 to 100 rpm, 30 min to 1 h). The resulting mixed solution is centrifuged (3,000 to 4,000 g, 10 min), and the supernatant after centrifugation is discarded to obtain a concentrated product of the sample.

Step (A2-a): A lysis buffer is added to the concentrated product under a condition of pH 7 to 8 to resuspend the wastewater sediment. A protease is added to the suspension, and the mixture is reacted by heating at 50 to 60°C to completely dissolve and lyse the wastewater sediment.

Step (A3): The nucleic acid in the concentrated product is bound to the solid carrier and separated. Examples of the separation of the nucleic acid from the solid carrier include a method in which the solid carrier is fixed to a magnet such as a magnetic table, the supernatant is washed with an organic solvent such as ethanol, and then dissolved and lysed with an inorganic solvent such as water, and the like.

Steps (B) and (C): Reverse transcription and pre-amplification are performed in the same manner as in steps (b1) and (c) of Method 2.

Step (D): qPCR is performed using the pre-amplification product as a template in the same manner as in step (d) of Method 1.

The present invention includes a kit for quantitatively detecting RNA and/or DNA derived from a virus or a bacterium in an environmental sample or a fecal sample, the kit comprising particularly high basicity PAC among PACs. Specifically, examples of the kit include a kit for quantitatively detecting RNA and/or DNA derived from a virus or a bacterium in an environmental sample or a fecal sample which contains high basicity PAC and is used in the method (III) of the present invention or the like. The kit may further include triiron tetraoxide. The kit may further include a buffer (lysis buffer) with a pH of 7 to 11 for dissolving and lysing wastewater sediment.

The present invention includes a method using magnetic beads, particularly carboxyl group-modified paramagnetic beads, as a solid carrier.

### (Method 5)

Step (A1-a): A concentrated product is obtained in the same manner as in Method 4. Step (A3-a): Carboxy-modified paramagnetic beads are further added to the suspension to bind and separate RNA and/or DNA in the suspension.

Steps (B) and (C): Reverse transcription and pre-amplification are performed in the same manner as in steps (b1) and (c) of Method 2.

Step (D): qPCR is performed using the pre-amplification product as a template in the same manner as in step (d) of Method 1.

### (Invention related to the method (IV) of the present invention)

The present invention also includes a method for concentrating an environmental sample or a fecal sample in the presence of high basicity PAC, dissolving and lysing the concentrated product of the sample under basic conditions, and separating a nucleic acid derived from a virus or a bacterium.

### (Method 6)

Step (A1-a): A concentrated product is obtained in the same manner as in step (A1-a) of Method 4.

Step (A2-b): RNA in the concentrated product is bound to the solid carrier in the same manner as in step (A2-a) of Method 4 except for under a condition of pH 8 to 11.

Step (A3): RNA on the solid carrier is separated in the same manner as in (A3) of Method 4.

Steps (B) and (C): Reverse transcription and pre-amplification are performed in the same manner as in steps (b1) and (c) of Method 2.

Step (D): qPCR is performed using the pre-amplification product as a template in the same manner as in step (d) of Method 1.

### (Invention related to the method (V) of the present invention)

The present invention includes a method for quantitatively detecting RNA and/or DNA derived from a virus or a bacterium in an environmental sample or a fecal sample, the method including the steps of: obtaining a concentrated product of the sample in the presence of PAC; dissolving and lysing the concentrated product under a condition of pH 7 to 11; and binding the RNA and/or DNA to a solid carrier and separating the RNA and/or DNA. The method (V) of the present invention includes the methods (III) and (IV) of the present invention.

In step (A1), a concentrated product of an environmental sample or a fecal sample is obtained in the presence of PAC. As a method for concentrating a sample, the same method as the method for concentrating an environmental sample or a fecal sample described in the above step (a) can be used.

For example, when a sample is concentrated by centrifugation, the method includes a method of standing the sample at room temperature (20 to 25°C) for 1 to 10 minutes or stirring the sample at 4°C for 10 minutes, and the like.

A preferred concentration of PAC per volume of environmental sample or fecal sample is 0.001 to 0.01% (w/v), and more preferably 0.001 to 0.005% (w/v).

PAC in the method (V) of the present invention includes normal PAC and high basicity PAC. The nucleic acid in the environmental sample or the fecal sample can be quantitatively detected regardless of the basicity of PAC, but the basicity is preferably 50 to 70% and more preferably 60% from Example 7. Examples thereof include the PAC as described in the method (II).

Triiron tetraoxide may be added as in the method (II) of the present invention.

In step (A2), the concentrated product obtained in step (A1) is dissolved and lysed under a condition of pH 7 to 11.

The pH of the lysis buffer is not particularly limited as long as it is the pH of a solution that can dissolve and lyse nucleic acids derived from a virus or bacterium, proteins derived from a virus or a bacterium, and contaminant proteins in wastewater contained in the wastewater sediment, but the pH of the lysis buffer is preferably 7 to 11, more preferably 7.5 to 9, and particularly preferably pH 8. In fact, in a case where a virus-derived nucleic acid in a sample is quantitatively detected using a lysis buffer at pH 8, a result has been obtained that the detection rate is higher than that in a case of using a lysis buffer having another pH, and the virus-derived nucleic acid can be more versatilely used even for various wastewater under different conditions.

Step (A2) can be performed in the same manner as step (A2-a) of the method (III) of the present invention except that it is under a condition of pH 7 to 11, and may or may not include the step of reacting a protease.

Steps (B), (C) and (D) can be performed in the same manner as the step of the method (III) of the present invention, and step (C) may or may not be carried out.

### (Method 7)

Step (A1): PAC is added to a wastewater sample (10 to 20 mL), and the mixture is mixed and then stirred (50 to 100 rpm, 30 min to 1 h). The resulting mixed solution is centrifuged (3,000 to 4,000 g, 10 min), and the supernatant after centrifugation is discarded to obtain a concentrated product of the sample.

Step (A2): A lysis buffer is added to the concentrated product under a condition of pH 7 to 11 to resuspend the wastewater sediment. A protease is added to the suspension, and the mixture is reacted by heating at 50 to 60°C to completely dissolve and lyse the wastewater sediment.

Steps (A3), (B), (C) and (D): These steps are performed in the same manner as in Method 4.

### (Method 8)

Step (A1-b): PAC is added to a wastewater sample (10 to 20 mL) so as to have a final concentration of 0.001 to 0.01% (w/v), and the mixture is mixed and then stirred (50 to 100 rpm, 30 min to 1 h). The resulting mixed solution is centrifuged (3,000 to 4,000 g, 10 min), and the supernatant after centrifugation is discarded to obtain a concentrated product of the sample.

Step (A2): This step is performed in the same manner as step (A2) of Method 7.

Step (A3-a): This step is performed in the same manner as step (A3-a) of Method 5.

Steps (B) and (C) and (D): These steps are performed in the same manner as in Method 4.

Further, the method (V) of the present invention includes a nucleic acid quantitative detection method in a sample which can be used when RNA and/or DNA derived from a virus or a bacterium is present in a relatively large amount in an environmental sample or a fecal sample. Specific examples thereof include a method not including the pre-amplification step of step (C) in (Method 4) to (Method 8) described above, and the like. An example will be shown below.

### (Method 9)

Steps (A1) and (A2): These steps are performed in the same manner as steps (A1) and (A2) of Method 7.

Step (A3): This step is performed in the same manner as in (A3) of Method 4.

Step (B): Reverse transcription is performed in the same manner as in step (b1) of Method 2.

Step (D): Quantitative PCR is performed using the cDNA obtained in step (B) and/or the DNA obtained in step (A3) as a template. QuantiTect Probe PCR Kit (QIAGEN) or the like can be used.

The present invention includes a kit for quantitatively detecting RNA and/or DNA derived from a virus or a bacterium in an environmental sample or a fecal sample, the kit comprising a PAC and a solid carrier. Preferably, examples of the kit include a kit for quantitatively detecting RNA and/or DNA derived from a virus or a bacterium in an environmental sample or a fecal sample used in the methods (III) to (V) of the present invention or the like.

As the PAC, a PAC usable in step (A1) of the method (V) of the present invention can be used. The amount of PAC included in the kit is not particularly limited, but a description such as "PAC is added to an environmental sample or a fecal sample so as to have a final concentration of 0.001 to 0.01% (w/v)" may be included in a protocol or the like attached to the kit.

As the solid carrier, a solid carrier usable in step (A3) of the method (III) of the present invention can be used. Preferred are magnetic beads, and particularly preferred are carboxyl group-modified paramagnetic beads.

The kit may further include a lysis buffer at pH 7 to 11 for dissolving and lysing wastewater sediment, and as the lysis buffer, a buffer usable in step (A2-a) of the method (III) of the present invention can be used.

It is fine if the lysis buffer is not included in the kit, and a composition of the lysis buffer is described in a protocol or the like attached to the kit, and a kit purchaser prepares the lysis buffer according to the protocol or the like.

The kit may further include a protease. The protease included in the kit is not limited, and examples thereof include proteinase K and the like.

The kit may further include triiron tetraoxide. The amount of triiron tetraoxide included in the kit is not limited, and for example, a description such as "0.01 to 1.0 g of triiron tetraoxide is added per 40 mL of an environmental sample or a fecal sample" may be included in a protocol or the like attached to the kit.

The kit may further include a reverse transcriptase kit, a quantitative PCR kit and/or a pre-amplification kit.

The reverse transcriptase kit is not particularly limited, but High Capacity cDNA Reverse Transcription Kit (ThermoFisher Scientific), iScript Advanced cDNA Synthesis Kit for RT-qPCR (Bio-Rad Laboratories, Inc.), Reliance Select cDNA Synthesis Kit (BIORAD) or the like can be used.

The quantitative PCR kit is not particularly limited, but TaqMan Environmental Master Mix 2.0 (ThermoFisher SCIENTIFIC), QuantiTect Probe PCR Kit (QIAGEN) or the like can be used.

The pre-amplification kit is not particularly limited, but BIOTAQ HS (Meridian BIOSCIENCE) or the like can be used.

Reverse transcription and pre-amplification can be performed by a single kit, and for example, iScript Explore One-Step RT and PreAmp Kit (Bio-Rad Laboratories, Inc.) or the like can be used.

### [EXAMPLES]

Hereinafter, the present invention will be explained in more detail by way of Examples of the present invention, but the present invention is not limited by them.

In the present specification, room temperature refers to 15 to 25°C.

### Example 1 Detection of SARS-CoV-2 from wastewater pellet (included in the method (I) of the present invention.)

Sediments obtained by crude centrifugation (5,000 g, 10 min) of several different wastewater were tested using the method described here. In addition, it has been found that this method is also effective for nucleic acid (RNA and/or DNA) from sludge. Specifically, using the method described in the present specification, RNA was extracted from wastewater and sludge collected in a wastewater treatment plant and a septic tank at a facility, RNA having a poly (A) sequence containing SARS-CoV-2 RNA at the 3' end was captured using a solid carrier on which Oligo dT₂₅ was immobilized, the solid carrier was subjected to a reverse transcription reaction, and then amplified (pre-amplified) by PCR, thereby generating an amplification product in a liquid phase using cDNA on the solid carrier as a template. A part of the amplification product generated in the liquid phase was subjected to quantitative analysis by quantitative PCR, thereby calculating the copy number of SARS-CoV-2 RNA in the sample.

The example below uses a vortex mechanical lysis process extraction and is incorporated to illustrate its use. This method uses carbide beads included in RNeasy PowerSoil Total RNA Kit, but it has been shown that the nucleic acid yield is not reduced by other beads such as glass beads.

### (Protocol)

1. Add up to 2 grams of sludge to a tube containing beads in the RNeasy PowerSoil Total RNA Kit.
2. Add 2.5 mL of solution PowerBeads, 250 µL of solution SR1, and 800 µL of IRS, to the sample.
3. Add 3.5 mL of phenol/chloroform/isoamyl alcohol to the sample.
4. Place the tube on a vortex adapter and vortex the tube on the highest setting for 1 minute.
5. Centrifuge the tube at 2500 g at room temperature for 10 minutes and collect 500 µL of the aqueous layer.
6. Charge 500 µL of Binding buffer into 500 µL of the aqueous layer, heat the mixture at 65°C for 5 minutes, and then rapidly cool the mixture at 4°C.
7. Add a total amount of 1 mL of the sample to magnetic beads with Oligo dT₂₅ suspended in 200 µL of Binding buffer in advance, and slowly stir the mixture with a rotator at room temperature for 5 minutes.
8. Fix the sample to a magnetic table, discard the supernatant, and wash the resulting sample with 500 µL of Wash buffer1.
9. Fix the sample to a magnetic table, discard the supernatant, and wash the resulting sample with 500 µL of Wash buffer1.
10. Fix the sample to a magnetic table, discard the supernatant, and wash the resulting sample with 500 µL of Low-salt buffer.
11. Discard all supernatant, suspend the sample in 10 µL of water, add the suspension to 10 µL of a mixed solution of reverse transcriptase prepared in advance, and perform heating at 25°C for 10 minutes, at 37°C for 120 minutes, and at 85°C for 5 minutes. The immobilized Oligo dT₂₅ functions as a primer.
12. Fix the sample to a strong magnet, discard the supernatant, suspend the sample in 10 µL of water, and add 10 µL of a bead suspension to a PCR reaction mix containing 40 µL of KOD one (TOYOBO) to make a total of 50 µL. Use a primer set of SARS-CoV-2-CDC-N1 (described in step 7 of RNA extraction protocol using novel Lysis buffer I of Example 3 described later).
13. Perform pre-amplification for 10 cycles at 98°C for 10 seconds, 55°C for 5 seconds, and 68°C for 5 seconds.
14. Subject the pre-amplification product to quantitative PCR (CDC-N1) in the same manner as step 8 of Example 3 described later, and calculate the copy number of N region of SARS-CoV-2 RNA (gene copy number/L).

RNA was extracted from a wastewater sample by the above method (in the present specification, described as the method (I)' of the present invention), and the results of detecting SARS-CoV-2 RNA are shown in Table 1. By analyzing the sample that was equal to or lower than the detection limit in the conventional method using PEG precipitation (see manual for detecting novel coronavirus genes in wastewater (COVID-19 Task Force of the Japan Society on Water Environment, Japan Institute of Wastewater Engineering and Technology, March 2021, [searched on April 5, 2021], Internet, <URL: https://www.jswe.or.jp/aboutus/pdf/SARS-CoV-2_RNA_Detection Manual_for_Wastewater.pdf>)) by the above method, as shown in Fig. 1, an equivalent amount of a sample that can be subjected to detection increased. As a result, the lower detection limit value decreased, and the sample was detected with Ct values of about 33 to 35.

**[Table 1]**

| Sample | Method | Ct value | Log (gene copy number/L) |
|---|---|---|---|
| A | Conventional method | Not detected | < 5.54 |
| | Method (I)' of present invention | 34.7 | 1.49 |
| B | Conventional method | Not detected | < 5.54 |
| | Method (I)' of present invention | 33.4 | 1.93 |

The reagents used are as follows.
· PowerBeads, SR1, IRS: RNeasy PowerSoil Total RNA Kit (QIAGEN) content
· Binding buffer: 100 mmol/L Tris-HCl, pH 7.5, 500 mmol/L LiCl, 0.5% lithium dodecyl sulfate (LiDS), 1 mmol/L EDTA, 5 mmol/L DTT
· Wash Buffer I: 20 mmol/L Tris-HCl, pH 7.5, 500 mmol/L LiCl, 0.1% LiDS, 1 mmol/L EDTA,5 mmol/L DTT
· Wash Buffer II: 20 mmol/L Tris-HCl, pH 7.5, 500 mmol/L LiCl, 1 mmol/L EDTA
· Low-Salt Buffer: 20 mmol/L Tris-HCl, pH 7.5, 200 mmol/L LiCl, 1 mmol/L EDTA

### Example 2 Concentration using magnetic flocculant, and RNA elution from aggregates with basic buffer, and detection of SARS-CoV-2

For the purpose of increasing the recovery rate of RNA in a wastewater sample so that RNA derived from a virus or a bacterium can be quantitatively detected even when the RNA is thinly contained in the sample, a step of obtaining a concentrated product from the sample was studied. As a result, it was found that the virus-derived RNA can be efficiently recovered from the wastewater sample by the following method (method (II)'of the present invention).

### (Protocol)

1. Add 0.02 g of triiron tetraoxide to 40 mL of wastewater, and stir the solution well.
2. Add 4 µL of PAC 300A (Taki Chemical Co., Ltd., basicity: 60%, aqueous solution of 10.5 wt% in terms of Al₂O₃ concentration) (final concentration 0.001% (w/v)) to the sample, and stir the solution well, and after 1 minute, stir the solution at a low speed to form flocs.
3. Fix the tube to a strong magnet and stand the tube for 1 minute.
4. Collect 140 µL of the supernatant with the tube fixed to the strong magnet, and then discard the supernatant.
5. Remove the tube from the strong magnet and suspend the tube in 500 µL of 0.5 M glycine buffer (pH 10.5).
6. Extract RNA from 140 µL of the suspension and 140 µL of the obtained supernatant using QIAamp Viral RNA Mini Kit (QIAGEN).
7. Add 10 µL of the RNA product to 10 µL of a mixed solution of reverse transcriptase prepared in advance, and perform heating at 25°C for 10 minutes, at 37°C for 120 minutes, and at 85°C for 5 minutes.
8. Subject pre reverse transcript to quantitative PCR (CDC-N1) in the same manner as step 8 of Example 3 described later, and measure the copy number of N region of SARS-CoV-2 RNA (gene copy number/L).

The results are shown in Fig. 2. The method of the present invention using PAC and triiron tetraoxide ("PAC+Fe₃O₄" in the figure) was performed according to the above protocol. The "direct extraction" method was performed by subjecting 40 mL of wastewater to 6 to 8 of the above protocol. The "crude centrifugation" method was performed by centrifuging at 3,100 g for 10 minutes, and subjecting the sediment after discarding the supernatant to 5 to 8 of the above protocol. The method using only "PAC" was performed by subjecting 40 mL of wastewater to 2 of the above protocol, then stirring at 60 rpm at 4°C for 30 minutes, centrifuging at 3,000 g, at 4°C for 10 minutes, and suspending the obtained sample in 500 µL of 0.5 M glycine buffer (pH 10.5) described in 5 of the above protocol, then subjecting the suspension to 6 to 8 of the above protocol.

By using PAC and triiron tetraoxide, the amount of viral RNA in the supernatant was reduced (that is, good aggregation efficiency) as compared with that by using crude centrifugation, and more RNA could be recovered from the sediment as compared with crude centrifugation. This method can efficiently recover not only a virus adsorbed on a solid but also a virus floating in the supernatant.

### Example 3 Study on extraction detection conditions of SARS-CoV-2RNA from wastewater sediment by protease treatment

In order to study extraction conditions of RNA derived from a virus or a bacterium contained in sediments obtained from wastewater collected from various points, RNA was extracted using various lysis buffer conditions or nucleic acid extraction methods described below, and the amount of extracted RNA was measured. As a lysis buffer used during dissolution and lysis, a buffer attached to RNeasy PowerSoil Total RNA Kit or novel Lysis buffer I was studied.

### (RNA extraction detection protocol using novel Lysis buffer I)

1. Add 1 × 10⁷ copies of inactivated SARS-CoV-2 based on the amount of RNA measured by quantitative PCR to a wastewater sediment obtained by crudely centrifuging (3,000 g, 10 min) 20 mL of wastewater (urban wastewater in wastewater treatment plant at point γ), and stand the mixture on ice for 30 minutes.
2. Add 570 µL of Lysis buffer I to the wastewater sediment obtained in step 1, and suspend the sediment.
3. Add 14 µL of proteinase K solution (20 mg/ml) at a final concentration of 0.5 mg/mL) to the suspension obtained in step 2, and allow the mixture to react under the following three conditions.

With protease treatment + condition 1: Stand the mixture at room temperature for 20 minutes.

With protease treatment + condition 2: Stand the mixture at 56°C for 10 minutes, and then stand the mixture at room temperature for 10 minutes.

With protease treatment + condition 3: Stand the mixture at 70°C for 5 minutes, and then stand the mixture at room temperature for 15 minutes.
4. Add 580 µL of phenol/chloroform/isoamyl alcohol (PCI solution; pH 5.2) to each sample obtained in step 3 or the sample obtained in step 2 (without protease treatment), and vortex the mixture at 3000 rpm for 1 minute. Centrifuge the resulting solution at 13,000 g for 10 minutes at room temperature, and collect 500 µL of the aqueous layer.
5. Purify 200 µL of the aqueous layer with NucleoSpin RNA Clean-up (Takara Bio Inc.), and elute the purified aqueous layer with 40 µL of water to obtain an RNA purified product.
6. Perform a reverse transcription reaction on 10 µL of the RNA purified product using High Capacity cDNA Reverse Transcription Kit (ThermoFisher Scientific) to synthesize cDNA.
7. Perform pre-amplification of the cDNA sample for 10 cycles using a SARS-CoV-2-CDC-N1 primer set (2019-nCoV_N1-F (5'-GACCCCAAAATCAGCGAAAT-3' (SEQ ID NO: 1)) and 2019-nCoV_N1-R (5'-TCTGGTTACTGCCAGTTGAATCTG-3' (SEQ ID NO: 2))) (Centers for Disease Control and Prevention, <URL:https://www.cdc.gov/coronavirus/2019-ncov/lab/rt-pcr-panel-primer-probes.html>) and KOD One (TOYOBO CO., LTD.) at 98°C for 10 seconds, 55°C for 5 seconds, and 68°C for 5 seconds.
8. Subject the sample obtained in step 7 to quantitative PCR using QuantiTect Probe PCR Kit (QIAGEN). Measure plasmid DNA dilution series containing SARS-CoV-2 positive control sequence (nCoV-N1-Control Plasmid (catalog number: 5004-N10001, eurofins)) in parallel by the primer set and probe of SARS-CoV-2-CDC-N1 (2019-nCoV_N1-P (5'-FAM-ACCCCGCATTACGTTTGGTGGACC-BHQ1-3' (SEQ ID NO: 3)), <URL:https://www.cdc.gov/coronavirus/2019-ncov/lab/rt-pcr-panel-primer-probes.html>), and calculate the copy number of N region of SARS-CoV-2 RNA (gene copy number/20 ml).

### (RNA extraction protocol using buffer attached to RNeasy PowerSoil Total RNA Kit)

1. Perform a step similar to step 1 of RNA extraction protocol using novel Lysis buffer I on 20 mL of wastewater (wastewater at point γ).
2. Add 400 µL of PowerBead Solution buffer attached to RNeasy PowerSoil Total RNA Kit to a wastewater sediment and suspend the mixture.
3. Add 40 µL of IRS buffer attached to RNeasy PowerSoil Total RNA Kit and 130 µL of IRS buffer, and suspend the mixture.
4-8. Perform a step similar to step 4-8 of RNA extraction protocol using novel Lysis buffer I.

**[Table 2]**

| Nucleic acid lysis buffer | Number of copies |
|---|---|
| Buffer attached to RNeasy PowerSoil Total RNA Kit (*Kit does not include protease) | 14621 |
| Lysis buffer I Without protease treatment | 38481 |
| Lysis buffer I With protease treatment + condition 1 | 51571 |
| Lysis buffer I With protease treatment + condition 2 | 57442 |
| Lysis buffer I With protease treatment + condition 3 | 45781 |

As described in Table 2, the numbers of copies of N region of SARS-CoV-2 RNA extracted and detected using Lysis Buffer I at pH 7.5 and the buffer attached to neutral Neasy PowerSoil Total RNA Kit were shown to be very large. Further, when proteinase K solution was added to Lysis Buffer I and heating treatment at 56°C was performed (condition 2), it was shown that the number of detected RNA copies was the largest.

From Example 3 and Examples 4 and 5 below, it is clear that the method for detecting viral RNA including a step of suspending wastewater pellet under a condition of pH 7 to 11 is an excellent method for quantitatively detecting nucleic acid in wastewater. Furthermore, it is clear that the method for detecting viral RNA, which includes protease treatment and heating treatment under a condition of pH 7 to 11 and includes a step of suspending wastewater sediment, is a very excellent method for quantitatively detecting nucleic acid in wastewater.

The reagents used are as follows.
· Lysis Buffer I: 100 mmol/L Tris-HCl pH 7.5, 100 mmol/L NaCl, 1% SDS, 10 mmol/L EDTA, 2 mmol/L DTT

When the pH was adjusted, the pH was adjusted using a trace amount of NaOH or HCl.

### Example 4 Comparative study on RNA purification methods using various magnetic beads (included in the methods (III) to (V) of the present invention.)

In order to improve the SARS-CoV-2 viral RNA recovery efficiency in wastewater by a flocculant and obtain conditions of the accompanying nucleic acid purification method, the following conditions A or B including the presence or absence of the flocculant and magnetic beads or columns capable of binding nucleic acid derived from a virus or a bacterium were studied using a sample obtained by adding inactivated SARS-CoV-2 virus to urban wastewater α.

### (Common Protocol 4-1)

1. Place in a centrifuge tube 20 mL of wastewater before centrifugation (urban wastewater at wastewater treatment plant at point γ) to which 1 × 10⁷ copies of inactivated SARS-CoV-2 have been added based on the amount of RNA measured by quantitative PCR. Obtain a sample to which PAC 300A is added as a flocculant and shaken (final concentration: 0.001% (w/v) or 0.005% (w/v)) or a sample to which no flocculant is added.
2. Stir each sample obtained in step 1 at 4°C, 60 rpm for 30 minutes, centrifuge the sample at 3,000 g, 10 minutes, 4°C, and completely discard the supernatant.
3. Add 570 µL of Lysis buffer (Lysis buffer I or Lysis buffer II), suspend the mixture well, and transfer the suspension to a 1.5 mL centrifuge tube.
4. Add 14 µL of proteinase K solution to the tube (final concentration: 0.5 mg/mL), suspend the mixture well, heat the suspension on a heat block at 56°C for 10 minutes, and then stand the suspension at room temperature for 5 minutes.
5. Add 580 µL of PCI solution (pH 5.2) to the tube, and suspend the solution by vortexing.
6. Stand the tube for 1 minute, and then centrifuge the tube at 13,000 g for 10 minutes at 15°C.
7. Take 500 µL of a supernatant aqueous layer of the tube to obtain a nucleic acid-containing crude extraction sample.

### (Condition A: Purification method using commercially available spin column kit)

A1. Purify 500 µL of the nucleic acid-containing crude extraction sample obtained according to Common Protocol 4-1 with NucleoSpin RNA Clean-up (Takara Bio Inc.), and elute the purified sample with 40 µL of water to obtain an RNA product.

### (Condition B: Purification method using magnetic beads (carboxy group-modified magnetic beads))

B1. Add 180 µL of RNAClean XP (BECKMAN COULTER) to 100 µL of the nucleic acid-containing crude extraction sample obtained according to Common Protocol 4-1, suspend the mixture by pipetting, and stand the suspension for 5 minutes.
B2. Fix the tube to a magnetic table, and stand the tube for 10 minutes.
B3. Discard the supernatant of the tube, and wash the tube with 70% ethanol.
B4. Discard the supernatant of the tube, and stand the tube for 15 minutes to be dried.
B5. Remove the tube from the magnetic table, suspend pellet with 30 µL of water, and stand the tube for 3 minutes.
B6. Fix the tube to the magnetic table, and collect and transfer the supernatant to a new 1.5 mL centrifuge tube to obtain an RNA sample.

### (Common Protocol 4-2)

1. Subject the RNA sample recovered in the step A1 or step B6 to a reverse transcription reaction in the same manner as in step 6 of Example 3 to synthesize cDNA.
2. Perform pre-amplification of the cDNA obtained in the step 1 in the same manner as in step 7 of Example 3.
3. Perform quantitative PCR of each pre-amplified sample obtained in the step 2 in the same manner as in step 8 of Example 3 and calculate the copy number of N region of SARS-CoV-2 RNA (gene copy number/20 ml).

The step of suspending the wastewater sediment in Lysis buffer I is included in step 3 of (Common Protocol 4-1), and the detected SARS-CoV-2RNA copy number (gene copy number/20 ml) is shown in Table 3.

**[Table 3]**

| PAC and Lysis buffer conditions | Condition A | Condition B |
|---|---|---|
| Lysis buffer I | 1045 | 1558 |
| PAC 0.001% + Lysis buffer I | 99664 | 84097 |

The step of suspending the wastewater sediment in Lysis buffer II is included in step 3 of (Common Protocol 4-1), and the detected SARS-CoV-2RNA copy number (gene copy number/20 ml) is shown in Table 4.

**[Table 4]**

| PAC and Lysis buffer conditions | Condition A | Condition B |
|---|---|---|
| PAC 0.001% + Lysis buffer II | 56023 | 77357 |
| PAC 0.005% + Lysis buffer II | 12924 | 27246 |

From the results in Table 3 and Table 4, it has been revealed that when a silica column under Condition A or carboxyl group-modified paramagnetic beads (RNAClean XP) (BECKMAN COULTER) under Condition B were used, SARS-CoV-2RNA could be detected regardless of the presence or absence of PAC.

Further, as a result of comparing the combination of the concentration of PAC as a flocculant and Lysis Buffer to be used, it has been revealed that SARS-CoV-2RNA 50 times or more as large can be detected under the condition of a final concentration of PAC of 0.001% and Lysis buffer I as compared with conditions not containing PAC.

It has been revealed that RNAClean XP (BECKMAN COULTER) can efficiently bind virus-derived nucleic acids in wastewater, can easily wash contaminants with magnetic manipulation and ethanol-containing water, and can recover nucleic acids with organic solvent-free water. That is, by using the carboxyl group-modified paramagnetic beads, it is possible to easily and efficiently recover and detect the nucleic acid in the wastewater sample.

Also, from the results in Table 4, it was found that even when the pH was under basic conditions, the efficiency was lower than that under neutral conditions, but the recovery rate of nucleic acid was increased.

The reagents used are as follows.
· Lysis Buffer I (pH 7.5): 100 mmol/L Tris-HCl, 100 mmol/L NaCl, 1% SDS, 10 mmol/L EDTA, 2 mmol/L DTT
· Lysis Buffer II (pH 10.6): 260 mmol/L glycine buffer, 200 mmol/L Tris-HCl, 200 mmol/L NaCl, 2% SDS, 20 mmol/L EDTA, 2 mmol/L DTT

### Example 5 Study on general-purpose RNA detection conditions from urban wastewater having a plurality of characteristics (included in the method (III) of the present invention.)

For the purpose of stably detecting SARS-CoV-2RNA even from a wastewater sample thinly containing RNA derived from a virus or a bacterium and a large amount of PCR inhibitory substances and the like, in the step of suspending wastewater sediment obtained from the sample, a composition of a lysis buffer that efficiently dissolves and lyses nucleic acids by dissolving and lysing proteins in the sediment and cleaving disulfide bonds, heating treatment, and the like were studied. In order to measure detection sensitivity in non-enveloped viruses, Pepper mild mottle virus (PMMoV), which is generally present in large amounts in wastewater and human feces, was also studied.

### (Protocol)

1. Place in a centrifuge tube 10 mL of urban wastewater at wastewater treatment facilities at three points α, β, or γ, add 1 µL of PAC 300 A, and shake the mixture (final concentration: 0.001% (w/v)).
2. Stir the tube at 4°C, 60 rpm for 30 minutes.
3. Centrifuge the tube at 3,000 g, 10 minutes, 4°C, and completely discard the supernatant.
4. Add 250 µL of any one of the following lysis buffers (Lysis Buffer I, Lysis Buffer III, Lysis Buffer IV, Lysis Buffer V), suspend the mixture well, and transfer the mixture to a 1.5 mL centrifuge tube.
5. Add 14 µL of proteinase K solution to the tube, suspend the suspension well, heat the suspension on a heat block at 56°C for 10 minutes, and then stand the suspension at room temperature for 5 minutes.
6. Add 300 µL of PCI solution (pH 5.2) to the tube, and suspend the solution by vortexing.
7. Stand the tube for 1 minute, and centrifuge the tube at 19,000 g for 10 minutes at 15°C.
8. Transfer 100 µL of the supernatant of the tube to another 1.5 mL centrifuge tube, add 180 µL of RNAClean XP (BECKMAN COULTER), and suspend the mixture well by pipetting for 5 minutes.
9. Fix the tube to a magnetic table, and stand the tube for 10 minutes.
10. Discard the supernatant of the tube, and wash the tube with 500µL of 70% ethanol.
11. Repeat the washing operation of 10. four times.
12. Discard the supernatant of the tube, and stand the tube for 15 minutes to be dried.
13. Remove the tube from the magnetic table, suspend pellet with 30 µL of water, and stand the tube for 3 minutes.
14. Fix the tube to the magnetic table, and collect and transfer the supernatant to a new 1.5 mL centrifuge tube to obtain an RNA sample.
15. Perform a reverse transcription reaction and 10 cycles of pre-amplification reaction on 1 µg of the RNA sample using iScript (trademark registration) Explore One-step RT and PreAmp kit (Bio-Rad) to synthesize cDNA.
16. Subject each cDNA obtained in step 15 to quantitative PCR using QuantiTect Probe PCR Kit (QIAGEN), SARS-CoV-2CDC-N1 or PMMoV primer set and probe, and calculate the copy number of N region of SARS-CoV-2 RNA or PMMoV RNA, in the same manner as in step 7 of Example 3. Use PMMV-FP1-rev (5'-GAGTGGTTTGACCTTAACGTTTGA-3' (SEQ ID NO: 4)) and PMMV-RP1 (5'-TTGTCGGTTGCAATGCAAGT-3' (SEQ ID NO: 5)) (Appl Environ Microbiol. 2013 Dec; 79(23): 7413-7418.) as the PMMoV primer sets. Use PMMV-Probe1 (5'-FAM-CCTACCGAAGCAAATG-MGB-NFQ-3' (SEQ ID NO: 6)) as the PMMoV probe. Measure plasmid DNA containing SARS-CoV-2 positive control sequence (nCoV-N1-Control Plasmid (catalog number: 5004-N10001, eurofins) and plasmid DNA dilution series containing PMMoV positive control sequence (5'-GAGTGGTTTGACCTTAACGTTTGAGAGGCCTACCGAAGCAAATGTCGCACTTGCAT TGCAACCGACAA-3' (SEQ ID NO: 7)) in parallel, and calculate the copy numbers of N region of SARS-CoV-2 RNA and PMMoV RNA.

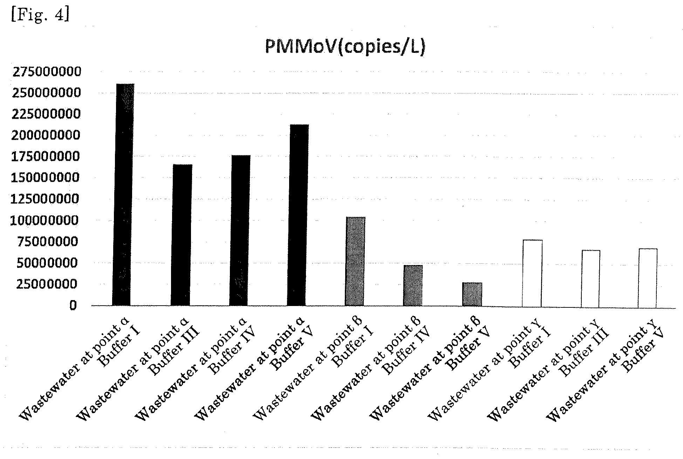

The observed concentrations (gene copy numbers/L) of SARS-CoV-2 RNA or the observed concentrations (gene copy numbers/L) of PMMoVRNA detected when the wastewater sediment was suspended by each Lysis buffer are shown in Fig. 3 or Fig. 4.

From Fig. 3 and Fig. 4, it has been revealed that RNA can be extracted and detected in a plurality of wastewater in a versatile manner when the wastewater sediment has been suspended using high basicity PAC as a flocculant and Lysis buffer I, III, IV or V with pH 7.5.

From Example 5, it was shown that the method using high basicity PAC and a lysis buffer at pH 7 to 8 has high versatility and excellent performance in quantitative detection of nucleic acid derived from not only an enveloped virus such as SARS-CoV-2 but also a non-enveloped virus such as PMMoV in a plurality of urban wastewater samples.

The reagents used are as follows.
· Lysis Buffer I (pH 7.5): 100 mmol/L Tris-HCl, 100 mmol/L NaCl, 1% SDS, 10 mmol/L EDTA, 2 mmol/L DTT
· Lysis Buffer III (pH 7.5): 200 mmol/L Tris-HCl, 200 mmol/L NaCl, 2% SDS, 20 mmol/L EDTA, 2 mmol/L DTT
· Lysis Buffer IV (pH 7.5): 100 mmol/L Tris-HCl, 100 mmol/L NaCl, 1% SDS, 10 mmol/L EDTA, 2% CTAB, 2 mmol/L DTT
· Lysis Buffer V (pH 7.5): 100 mmol/L Tris-HCl, 2 M NaCl, 20 mmol/L EDTA, 2% CTAB, 2 mmol/L DTT

### Example 6 Study on SARS-CoV-2RNA extraction efficiency and detection efficiency for a plurality of urban wastewater sampled over time (included in the method (III) of the present invention.)

SARS-CoV-2 and PMMoV were detected from wastewater sediment obtained from wastewater collected at a plurality of points and consecutive time points using a PAC flocculant and a lysis buffer at pH 7.5. The quantitative detection efficiency was compared with the detection and quantitative efficiency of viral RNA by a general detection method using a commercial kit for detection of viral RNA derived from wastewater or feces.

### (Protocol for viral RNA detection method using PAC flocculant and lysis buffer at pH 7.5) (in the present specification, it is referred to as the method (III)' of the present invention.)

Collect 10 mL of urban wastewater at wastewater treatment facilities at point D or point E in six schedules, and quantitatively detect SARS-CoV-2RNA in the same manner as in Example 5. Use Lysis Buffer I in a step corresponding to step 4 of Example 5, use iScript Advanced cDNA Synthesis Kit for RT-qPCR (Bio-Rad Laboratories, Inc.) and BIOTAQ HS (meridian BIOSCIENCE) in a step corresponding to step 15 of Example 5, and use TaqMan (registered trademark) Environmental Master Mix 2.0 (ThermoFisher SCIENTIFIC) in a step corresponding to step 16 of Example 5.

### (Protocol for viral RNA detection method using commercial kit)

1. Collect 40 mL of urban wastewater at wastewater treatment facilities at two points α or 6 in six schedules, place the urban wastewater in a centrifuge tube, centrifuge the tube at 6800 g, 10 min, 4°C, and discard the supernatant.
2. Suspend the resulting product in 650 µL of the attached PM1 buffer using RNeasy PowerMicrobiome Kit (QIAGEN).
3. Add 100 µL of TRIzol RNA Isolation Reagents (Thermofisher scientific) to the suspension obtained in step 2, and mix and stir the mixture at 3000 rpm for 10 minutes.
4. Centrifuge the tube of the above 3 at 13,000 g for 1 minute at room temperature.
5. Elute RNA in a volume of 50 µL above according to the protocol attached to the PowerMicrobiome Kit using 450 µL of the supernatant obtained from the tube of 4.
6. Perform a reverse transcription reaction and pre-amplification on 1 µg of the RNA obtained in the above 5 in the same manner as in Example 3 using iScript (registered trademark) Explore One-step RT and PreAmp kit.
7. Subject the pre-amplified sample to quantitative PCR using Quantitect Probe Mix. Calculate the SARS-CoV-2RNA concentration (copy/L) in the same manner as in Example 3.

The observed concentrations (gene copy numbers/L) of SARS-CoV-2 RNA detected by extracting RNA from a wastewater sample by the method (III) of the present invention or a method using a commercial kit are shown in Tables 5 and 6.

**[Table 5]**

| | Method (III)' of present invention | Method using a commercial kit |
|---|---|---|
| 2021/7/21 Wastewater at point α | 472875 | 5093 |
| 2021/7/26 Wastewater at point α | 116642 | 3056 |
| 2021/7/28 Wastewater at point α | 34759 | 2685 |
| 2021/7/30 Wastewater at point α | 22197 | 6111 |
| 2021/8/2 Wastewater at point α | 175997 | 8241 |
| 2021/8/4 Wastewater at point α | 337970 | 2870 |

**[Table 6]**

| | Method (III)' of present invention | Method using a commercial kit |
|---|---|---|
| 2021/7/21 Wastewater at point β | 4760 | 5556 |
| 2021/7/26 Wastewater at point β | 12834 | 3241 |
| 2021/7/28 Wastewater at point β | 8776 | 2593 |
| 2021/7/30 Wastewater at point β | 94675 | 8611 |
| 2021/8/2 Wastewater at point β | 47005 | 7222 |
| 2021/8/4 Wastewater at point β | 116687 | 833 |

As shown in Tables 5 and 6, it was suggested that the RNA extraction method using high basicity PAC and lysis buffer at pH 7.5 can quantitatively detect 37 times or more of SARS-CoV-2 viral RNA on average, as compared with the method using a commercial kit when detecting viral RNA derived from wastewater or feces. Furthermore, it was suggested that the method using high basicity PAC and a lysis buffer at pH 7 to 8 can efficiently and quantitatively detect nucleic acids contained in wastewater under different environments.

### Example 7 Comparative study on RNA purification using PACs with different basicity (included in the methods (III) to (V) of the present invention.)

It is known that characteristics of PAC vary depending on various basicities. In order to clarify whether the basicity of PAC affects SARS-CoV-2 RNA recovery efficiency, a detection experiment of added virus was performed using PAC flocculants having different basicities, using a sample obtained by adding inactivated SARS-CoV-2 virus to urban wastewater.

1. Place in a centrifuge tube 10 mL of urban wastewater before centrifugation (urban wastewater at wastewater treatment plant at point δ) to which 1 × 10⁶ copies of inactivated SARS-CoV-2 have been added based on the amount of RNA measured by quantitative PCR. Obtain a sample to which 1 µl of PAC 250A (Taki Chemical Co., Ltd., basicity: 50%, aqueous solution of 10.5 wt% in terms of Al₂O₃ concentration), PAC 300A (Taki Chemical Co., Ltd., basicity: 60%, aqueous solution of 10.5 wt% in terms of Al₂O₃ concentration) or PAC 700A (Taki Chemical Co., Ltd., basicity: 70%, aqueous solution of 10.5 wt% in terms of Al₂O₃ concentration) (final concentration: 0.001% (w/v)) is add as a flocculant and shaken, or a sample shaken without adding a flocculant.
2. Stand each sample obtained in step 1 at 20°C for 10 minutes, centrifuge the sample at 3,000 g, 10 min, 4°C, and completely discard the supernatant.
3. Add 250 µL of Lysis buffer I (pH 7.5), suspend the mixture well, and transfer the suspension to a 1.5 mL centrifuge tube.
4. Add 14.25 µL of proteinase K solution to the tube (final concentration: 1.14 mg/mL or more), suspend the mixture well, heat the suspension on a heat block at 56°C for 10 minutes, and then stand the suspension at room temperature for 5 minutes.
5. Add 550 µL of PCI solution (pH 5.2) to the tube, and suspend the solution by vortexing.
6. Stand the tube for 1 minute, and centrifuge the tube at 19,000 g for 10 minutes at 20°C.
7. Take 200 µL of a supernatant aqueous layer of the tube to obtain a nucleic acid-containing crude extraction sample.
8. Add 180 µL of RNAClean XP (BECKMAN COULTER) to 100 µL of the nucleic acid-containing crude extraction sample obtained according to Common Protocol, suspend the mixture by pipetting, and stand the suspension for 5 minutes.
9. Fix the tube to a magnetic table, and stand the tube for 10 minutes.
10. Discard the supernatant of the tube, wash the tube with 70% ethanol, and repeat step 10 a total of 5 times.
11. Discard the supernatant of the tube, and stand the tube for 15 minutes to be dried.
12. Remove the tube from the magnetic table, suspend pellet with 30 µL of water, and stand the tube for 3 minutes.
13. Fix the tube to the magnetic table, and collect and transfer the supernatant to a new centrifuge tube to obtain an RNA sample.
14. Perform a reverse transcription reaction using 1 µg of the RNA sample recovered in step 13 and 2019-nCoV_N1-R using Reliance Select cDNA Synthesis Kit (Bio-Rad Laboratories, Inc.) to synthesize cDNA.
15. Perform 10 cycles of pre-amplification using the cDNA obtained in step 14 and plasmid DNA dilution series containing SARS-CoV-2 positive control sequence for copy number calculation (nCoV-N1-Control Plasmid (catalog number: 5004-N10001, eurofins), SARS-CoV-2CDC-N1 primer set and BIOTAQ HS (Meridian BIOSCIENCE).
16. Measure the pre-amplified sample obtained in step 15 using SARS-CoV-2CDC-N1 primer and probe using TaqMan Environmental Master Mix 2.0 (ThermoFisher SCIENTIFIC), and calculate the copy number (gene copy number/10 ml) of N region of SARS-CoV-2 RNA.

**[Table 7]**

| PAC | |
|---|---|
| PAC (-) | 14171 |
| PAC (basicity 50%) | 211564 |
| PAC (basicity 60%) | 252068 |
| PAC (basicity 70%) | 228485 |

The calculated copy number (gene copy number/10 ml) of N region of SARS-CoV-2 RNA is shown in Table 7. It was confirmed that, also in urban wastewater δ, the detection amount of SARS-CoV-2 virus RNA was improved by the addition of PAC flocculant. Furthermore, the detection amount was high even when using any of PACs with basicity of 50%, 60%, and 70%.

### Example 8 Comparative study on pH conditions during dissolution and lysis of wastewater sediment (included in the methods (III) to (V) of the present invention.)

In order to clarify optimum pH conditions for improving the quantitative detection efficiency of SARS-CoV-2 virus RNA, a detection experiment of added virus was performed using a sample obtained by adding inactivated SARS-CoV-2 virus to urban wastewater δ.
1. Place in a centrifuge tube 10 mL of wastewater before centrifugation (urban wastewater at wastewater treatment plant at point δ) to which 1 × 10⁵ copies of inactivated SARS-CoV-2 have been added based on the amount of RNA measured by quantitative PCR. Obtain a sample to which 1 µl of PAC 300A is added as a flocculant and shaken (final concentration: 0.001% (w/v)).
2. Stir each sample obtained in step 1 at 4°C, 60 rpm for 10 minutes, centrifuge the sample at 3,000 g, 10 minutes, 4°C, and completely discard the supernatant.
3. Add 250 µL of a buffer in which the pH of Lysis buffer I has been adjusted to pH 6, 7.5, 8, 9, 10 or 11, suspend the mixture well, and transfer the suspension to a 1.5 mL centrifuge tube.
4. Add 14.25 µL of proteinase K solution to the tube (final concentration: 1.07 mg/mL or more), suspend the mixture well, heat the suspension on a heat block at 56°C for 10 minutes, and then stand the suspension at room temperature for 5 minutes.
5. Add 550 µL of PCI solution (pH 5.2) to the tube, and suspend the solution by vortexing.
6. Stand the tube for 1 minute, and centrifuge the tube at 19,000 g for 10 minutes at 20°C.
7. Take 200 µL of a supernatant aqueous layer of the tube to obtain a nucleic acid-containing crude extraction sample.
8. Add 180 µL of RNAClean XP (BECKMAN COULTER) to 100 µL of the nucleic acid-containing crude extraction sample obtained in step 7, suspend the mixture by pipetting, and stand the suspension for 5 minutes.
9. Fix the tube to a magnetic table, and stand the tube for 10 minutes.
10. Discard the supernatant of the tube, and wash the tube with 70% ethanol.
11. Discard the supernatant of the tube, and stand the tube for 15 minutes to be dried.
12. Remove the tube from the magnetic table, suspend pellet with 30 µL of water, and stand the tube for 3 minutes.
13. Fix the tube to the magnetic table, and collect and transfer the supernatant to a new 1.5 mL centrifuge tube to obtain an RNA sample.
14. Perform a reverse transcription reaction using 1 µg of the RNA sample recovered in step 13 and 2019-nCoV_N1-R and PMMoV reverse primer using Reliance Select cDNA Synthesis Kit (Bio-Rad Laboratories, Inc.) to synthesize cDNA.
15. Obtain 10 cycles of pre-amplified samples using the cDNA obtained in step 14 and the plasmid DNA dilution series containing SARS-CoV-2 positive control sequence or PMMoV positive control sequence for copy number calculation, the SARS-CoV-2CDC-N1 primer and the PMMoV primer set and BIOTAQ HS (Meridian BIOSCIENCE). Furthermore, at the same time, perform 10 cycles of heat treatment for pre-amplification using the cDNA obtained in step 14 and the plasmid DNA dilution series containing PMMoV positive control sequence for copy number calculation, only the PMMoV reverse primer (PMMV-RP1), and BIOTAQ HS (Meridian BIOSCIENCE) to obtain a non-pre-amplified sample in which the cDNA fragment of PMMoV itself has not been pre-amplified.
16. Measure the SARS-CoV-2 pre-amplified and PMMoV non-pre-amplified samples obtained in step 15 using the SARS-CoV-2CDC-N1 primer and PMMoV primer set and probe, and TaqMan Environmental Master Mix 2.0 (ThermoFisher SCIENTIFIC), and calculate the copy number (gene copy number/10 ml) of N region of SARS-CoV-2 RNA and PMMoV RNA.

**[Table 8]**

| pH | SARS-CoV-2 | PMMoV |
|---|---|---|
| pH 6.0 | 46118 | 658479 |
| pH 7.5 | 92569 | 1339758 |
| pH 8.0 | 64236 | 1171102 |
| pH 9.0 | 126670 | 1306474 |
| pH 10.0 | 71178 | 1269888 |
| pH 11.0 | 58813 | 1036999 |

The calculated copy numbers of N region of SARS-CoV-2 RNA and PMMoV are shown in Table 8. From the present results, it has been revealed that when wastewater sediment is dissolved and lysed under a condition of pH 7.5 to pH 11.0, SARS-CoV-2 RNA and PMMoV RNA can be quantitatively detected more efficiently than under a condition of pH 6.0.

### [INDUSTRIAL APPLICABILITY]

The method of the present invention is a method for quantitatively detecting RNA and/or DNA derived from a virus or a bacterium in an environmental sample or a fecal sample with high sensitivity.

## Claims

1. A method for quantitatively detecting RNA and/or DNA derived from a virus or a bacterium in an environmental sample or a fecal sample, the method comprising the steps of:
(A1) obtaining a concentrated product of the sample in the presence of polyaluminum chloride;
(A2) dissolving and lysing the concentrated product under a condition of pH 7 to 11;
(A3) binding RNA and/or DNA in the concentrated product to a solid carrier and separating the RNA and/or DNA; and
(B) when RNA is separated in step (A3), performing reverse transcription using the RNA as a template to obtain cDNA,
wherein the method optionally comprises a step of
(C) performing pre-amplification using the cDNA obtained in step (B) and/or the DNA obtained in step (A3) as a template to obtain an amplification product in a liquid phase.

2. A method for quantitatively detecting RNA and/or DNA derived from a virus or a bacterium in an environmental sample or a fecal sample, the method comprising the steps of:
(a) extracting RNA and/or DNA from the sample or a concentrated product of the sample;
(b1) performing reverse transcription using the RNA hybridized to an oligonucleotide on a solid carrier as a template to obtain an immobilized cDNA, and/or
(b2) hybridizing the DNA to an oligonucleotide on a solid carrier; and
(c) performing pre-amplification using the cDNA and/or DNA on the solid carrier as a template to obtain an amplification product in a liquid phase.

3. The method according to claim 1 or 2, wherein the environmental sample is a wastewater sample, a water sample, an air sample, or a sample obtained from a solid surface.

4. The method according to any of claims 1 to 3, wherein the number of cycles of pre-amplification is 1 to 14 cycles.

5. The method according to any of claims 2 to 4, wherein the oligonucleotide is an oligonucleotide having an oligo (dT) sequence or a base sequence specific to a viral or bacterial nucleic acid to be detected.

6. The method according to any of claims 1 to 5, wherein the solid carrier is magnetic beads.

7. The method according to any of claims 1 to 6, wherein the virus is an RNA virus.

8. The method according to any of claims 1 to 7, wherein the virus is a coronavirus, an influenza virus, a norovirus, sapovirus, an Aichi virus, an adenovirus, an orthopneumovirus, or a polyomavirus.

9. The method according to claim 8, wherein the coronavirus is SARS-CoV-2.

10. The method according to any of claims 2 to 9, comprising a step of (d) quantifying the cDNA and/or DNA on the solid carrier by quantitative PCR using the amplification product obtained in step (c) as a template.

11. The method according to any of claims 1, 3, 4, and 6 to 9, comprising a step of (D) quantifying the cDNA and/or DNA by quantitative PCR using the amplification product obtained in step (C) or the cDNA obtained in step (B) and/or the DNA obtained in step (A3) as a template.

12. The method according to claim 10, wherein a Ct value of quantitative PCR using the amplification product as a template is 18 to 35.

13. The method according to any of claims 2 to 10 or 12, comprising a step of obtaining the concentrated product of step (a) from the environmental sample or the fecal sample using polyaluminum chloride and/or triiron tetraoxide.

14. The method according to any of claims 1, 3, 4, 6 to 9 or 11, wherein the polyaluminum chloride has a final concentration of 0.001 to 0.01 mass/volume percent in step (A1).

15. The method according to any of claims 1, 3, 4, 6 to 9, 11, or 14, wherein the polyaluminum chloride has a basicity of 50 to 70%.

16. The method according to any of claims 1, 3, 4, 6 to 9, 11, 14 or 15, wherein the step (A2) is performed under a condition of pH 7 to 8.

17. The method according to any of claims 1, 3, 4, 6 to 9, 11, or 14 to 16, wherein the step (A2) includes a step of reacting a protease.

18. A kit for quantitatively detecting RNA and/or DNA derived from a virus or a bacterium in an environmental sample or a fecal sample, the kit comprising polyaluminum chloride and a solid carrier.

19. The kit according to claim 18, wherein the solid carrier is carboxyl group-modified paramagnetic beads.
